(19) **Europäisches Patentamt**
**European Patent Office**
**Office européen des brevets**

(11) **EP 1 975 229 A2**

(12) # EUROPEAN PATENT APPLICATION

(43) Date of publication:
**01.10.2008 Bulletin 2008/40**

(51) Int Cl.:
**C12N 9/28** *(2006.01)* **C11D 3/386** *(2006.01)*
**D06L 1/14** *(2006.01)*

(21) Application number: **08156819.8**

(22) Date of filing: **12.10.2001**

(84) Designated Contracting States:
**AT BE CH CY DE DK ES FI FR GB GR IE IT LI LU MC NL PT SE TR**

(30) Priority: **13.10.2000 DK 200001533**
**02.10.2001 DK 200101442**

(62) Document number(s) of the earlier application(s) in accordance with Art. 76 EPC:
**01978207.7 / 1 326 965**

(71) Applicant: **Novozymes A/S**
**2880 Bagsvaerd (DK)**

(72) Inventor: **Andersen, Carsten**
**3500 Värlöse (DK)**

Remarks:
This application was filed on 23-05-2008 as a divisional application to the application mentioned under INID code 62.

(54) **Alpha-amylase variant with altered properties**

(57) The present invention relates to variants of parent alpha-amylases, which variant has alpha-amylase activity and exhibits an alteration in at least one of the following properties relative to said parent alpha-amylase: substrate specificity, substrate binding, substrate cleavage pattern, thermal stability, pH/activity profile, pH/stability profile, stability towards oxidation, specific activity, and altered pl, in particular higher pl.

**EP 1 975 229 A2**

**Description**

**FIELD OF THE INVENTION**

The present invention relates to variants (mutants) of parent alpha-amylases, in particular of *Bacillus* origin, which variant has alpha-amylase activity and exhibits an alteration in at least one of the following properties relative to said parent alpha-amylase: substrate specificity, substrate binding, substrate cleavage pattern, thermal stability, pH/activity profile, pH/stability profile, stability towards oxidation, specific activity, and pl, in particular higher pl.

**BACKGROUND OF THE INVENTION**

Alpha-Amylases (alpha-1,4-glucan-4-glucanohydrolases, E.C. 3.2.1.1) constitute a group of enzymes, which catalyze hydrolysis of starch and other linear and branched 1,4-glucosidic oligo- and polysaccharides.
The object of the invention is to provide an improved alpha-amylase, in particular suitable for detergent use.

**BRIEF DISCLOSURE OF THE INVENTION**

The object of the present invention is to provide an alpha-amylases which variants in comparison to the corresponding parent alpha-amylase, i.e., un-mutated alpha-amylase, has alpha-amylase activity and exhibits an alteration in at least one of the following properties relative to said parent alpha-amylase: substrate specificity, substrate binding, substrate cleavage pattern, thermal stability, pH/activity profile, pH/stability profile, stability towards oxidation, $Ca^{2+}$ dependency, specific activity, and pl.

Nomenclature

In the present description and claims, the conventional one-letter and three-letter codes for amino acid residues are used. For ease of reference, alpha-amylase variants of the invention are described by use of the following nomenclature:
Original amino acid(s): position(s): substituted amino acid(s)
According to this nomenclature, for instance the substitution of alanine for asparagine in position 30 is shown as:
Ala30Asn or A30N
a deletion of alanine in the same position is shown as:
Ala30* or A30*
and insertion of an additional amino acid residue, such as lysine, is shown as:
Ala30AlaLys or A30AK
A deletion of a consecutive stretch of amino acid residues, such as amino acid residues 30-33, is indicated as (30-33)* or Δ(A30-N33).
Where a specific alpha-amylase contains a "deletion" in comparison with other alpha-amylases and an insertion is made in such a position this is indicated as:
*36Asp or *36D
for insertion of an aspartic acid in position 36.
Multiple mutations are separated by plus signs, i.e.:
Ala30Asp + Glu34Ser or A30N+E34S
representing mutations in positions 30 and 34 substituting alanine and glutamic acid for asparagine and serine, respectively.
When one or more alternative amino acid residues may be inserted in a given position it is indicated as
A30N,E or
A30N or A30E
Furthermore, when a position suitable for modification is identified herein without any specific modification being suggested, it is to be understood that any amino acid residue may be substituted for the amino acid residue present in the position. Thus, for instance, when a modification of an alanine in position 30 is mentioned, but not specified, it is to be understood that the alanine may be deleted or substituted for any other amino acid, i.e., any one of:
R,N,D,A,C,Q,E,G,H,I,L,K,M,F,P,S,T,W,Y,V.
Further, "A30X" means any one of the following substitutions:
A30R, A30N, A30D, A30C, A30Q, A30E, A30G, A30H, A30I, A30L, A30K, A30M, A30F, A30P, A30S, A30T, A30W, A30Y, or A30 V; or in short: A30R,N,D,C,Q,E,G,H,I,L,K,M,F,P,S,T,W,Y,V.

**BRIEF DESCRIPTION OF THE DRAWINGS**

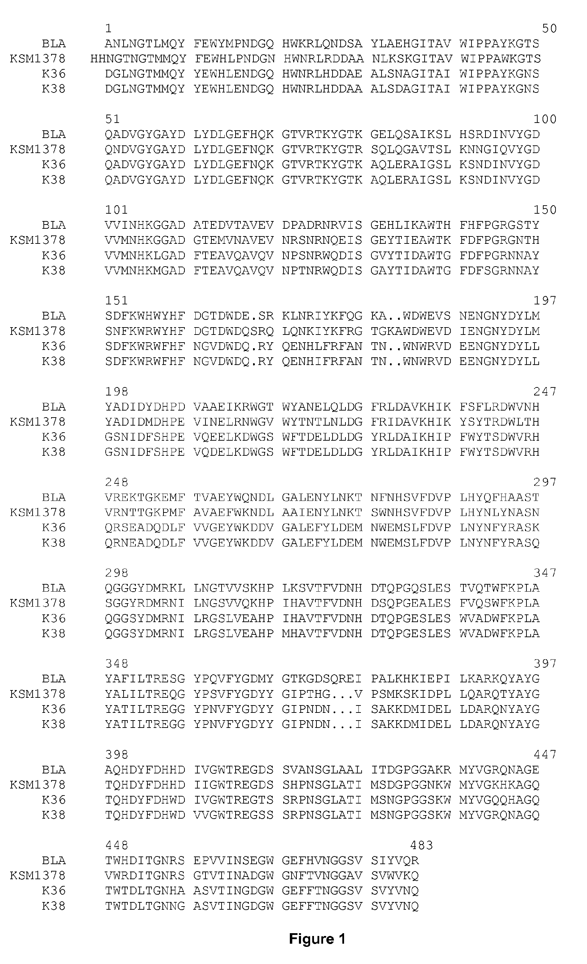

Figure 1 is an alignment of the amino acid sequences of five parent alpha-amylases. The numbers on the extreme left designate the respective amino acid sequences as follows:

1: SEQ ID NO: 6 *(Bacillus licheniformis* alpha-amylase)
2: SEQ ID NO: 8 (KSM-AP1378 alpha-amylase)
3: SEQ ID NO: 2 (KSM-K36 alpha-amylase)
4: SEQ ID NO: 4 (KSM-K38 alpha-amylase)

**DETAILED DISCLOSURE OF THE INVENTION**

The object of the present invention is to provide an alpha-amylases, in particular of *Bacillus* origin, which variants has alpha-amylase activity and exhibits an alteration in at least one of the following properties relative to said parent alpha-amylase: substrate specificity, substrate binding, substrate cleavage pattern, thermal stability, pH/activity profile, pH/stability profile, stability towards oxidation, specific activity, and altered pl, in particular higher pl.

Parent Alpha-amylases

Contemplated alpha-amylases include the alpha-amylases shown in SEQ ID NO: 2 or SEQ ID NO: 4 of *Bacillus* origin and alpha-amylase having at least 70% identity thereto. The SEQ ID NO: 1 shows the DNA sequence encoding KSM-K36 and SEQ ID NO: 3 show the DNA sequence encoding KSM-K38. These two alpha-amylases are disclosed in EP 1,022,334 (hereby incorporated by reference).
The KSM-K38 alpha-amylase has about 67% identity with the KSM-AP1378 alpha-amylase disclosed in WO 97/00324); 64% identity with the #707 alpha-amylase derived from *Bacillus* sp.#707 disclosed by Tsukamoto et al., Biochemical and Biophysical Research Communications, 151 (1988), pp. 25-31; and about 63% identity with the *Bacillius licheniformis* alpha-amylase described in EP 0252666 (ATCC 27811).
Other alpha-amylases within the scope of the present invention include alpha-amylases i) which displays at least 70%, such as at least 75%, or at least 80%, at least 85%, at least 90%, at least 95%, at least 97%, at least 99% homology with at least one of said amino acid sequences shown in SEQ ID NOS: 2 or 4, and/or ii) is encoded by a DNA sequence which hybridizes to the DNA sequences encoding the above-specified alpha-amylases which are apparent from SEQ ID NOS: 1 or 3.
In connection with property i), the homology may be determined as the degree of identity between the two sequences indicating a derivation of the first sequence from the second. The homology may suitably be determined by means of computer programs known in the art such as GAP provided in the GCG program package (described above). Thus, Gap GCGv8 may be used with the following default parameters: GAP creation penalty of 5.0 and GAP extension penalty of 0.3, default scoring matrix. GAP uses the method of Needleman/Wunsch/Sellers to make alignments.
Alternatively, the software Clustal X obtainable from EMBL (ftp.embl-heidelberg.de) may be used for multiple alignments with a gap creation penalty of 30, a gap extension penalty of 1 without gap penalty.
A structural alignment between the KSM-K36 or KSM-K38 alpha-amylases (SEQ ID NO: 2 and 4) and other alpha-amylase may be used to identify equivalent/corresponding positions in other alpha-amylases. One method of obtaining said structural alignment is to use the Pile Up programme from the GCG package using default values of gap penalties, i.e., a gap creation penalty of 3.0 and gap extension penalty of 0.1. Other structural alignment methods include the hydrophobic cluster analysis (Gaboriaud et al., (1987), FEBS LETTERS 224, pp. 149-155) and reverse threading (Huber, T; Torda, AE, PROTEIN SCIENCE Vol. 7, No. 1 pp. 142-149 (1998). An alignment of the KSM-K36, KSM-K38, KSM-AP1378 and the *Bacillus licheniformis* alpha-amylase is shown in Fig. 1.

Hybridisation

The oligonucleotide probe used in the characterisation of the KSM-K36 or KSM-K38 alpha-amylases in accordance with property ii) above may suitably be prepared on the basis of the full or partial nucleotide or amino acid sequence of the alpha-amylase in question.
Suitable conditions for testing hybridisation involve pre-soaking in 5xSSC and prehybridizing for 1 hour at ~40°C in a solution of 20% formamide, 5xDenhardt's solution, 50mM sodium phosphate, pH 6.8, and 50mg of denatured sonicated calf thymus DNA, followed by hybridisation in the same solution supplemented with 100mM ATP for 18 hours at 40°C, followed by three times washing of the filter in 2xSSC, 0.2% SDS at 40°C for 30 minutes (low stringency), preferred at 50°C (medium stringency), more preferably at 65°C (high stringency), even more preferably at 75°C (very high stringency). More details about the hybridisation method can be found in Sambrook et al., Molecular_Cloning: A Laboratory Manual, 2nd Ed., Cold Spring Harbor, 1989.

In the present context, "derived from" is intended not only to indicate an alpha-amylase produced or producible by a strain of the organism in question, but also an alpha-amylase encoded by a DNA sequence isolated from such strain and produced in a host organism transformed with said DNA sequence. Finally, the term is intended to indicate an alpha-amylase, which is encoded by a DNA sequence of synthetic and/or cDNA origin and which has the identifying characteristics of the alpha-amylase in question. The term is also intended to indicate that the parent alpha-amylase may be a variant of a naturally occurring alpha-amylase, i.e., a variant, which is the result of a modification (insertion, substitution, deletion) of one or more amino acid residues of the naturally occurring alpha-amylase.

Altered Properties

The following discusses the relationship between mutations, which are present in variants of the invention, and desirable alterations in properties (relative to those a parent KSM-K36 or KSM-K38 alpha-amylases), which may result therefrom.

As mentioned above the invention relates to alpha-amylase variants with altered properties.

In an aspect the invention relates to variant with altered properties as mentioned above.

In the first aspect a variant of a parent KSM-K36 or KSM-K38 alpha-amylase, comprising an alteration at one or more positions (using SEQ ID NO: 2 or 4 for the amino acid numbering) selected from the group of:
2,9,14,15,16,26,27,48,49,51,52,53,54,58,73,88,94,96,103,104,107,108,111,114,128,130,133,138 , 140,142,144,148, 149,156,161,165,166,168,171,173,174,178,179,180,181,183,184,187,188,190 , 194,197,198,199,200,201,202,203, 204,205,207,209,210,211,212,214,221,222,224,228,230,233 , 234,237,239,241,242,252,253,254,255,260,264,265, 267,275,276,277,280,281,286,290,293,301 , 305,314,315,318,329,333,340,341,356,375,376,377,380,383,384,386, 389,399,403,404,405,406 ,427,441,444,453,454,472,479,480
wherein

(a) the alteration(s) are independently

(i) an insertion of an amino acid downstream of the amino acid which occupies the position,
(ii) a deletion of the amino acid which occupies the position, or
(iii) a substitution of the amino acid which occupies the position with a different amino acid, (b) the variant has alpha-amylase activity and (c) each position corresponds to a position of the amino acid sequence of the parent alpha-amylase having the amino acid sequence of the KSM-K36 alpha-amylases shown in SEQ ID NO: 2.

In the KSM-38 alpha-amylase the target positions are: 2,9,14,15,16,26,27,48,49,51,52,53,54,58,73,88,94,96, 103,104,107,108,111,114,128,130,133,138 , 140,142,144,148,149,156,161,165,166,168,171,173,174,178,179,180, 181,183,184,187,188,190 , 194,197,198,199,200,201,202,203,204,205,207,209,210,211,212,214,221,222,224,228, 230,233 , 234,237,239,241,242,252,253,254,255,260,264,265,267,275,276,277,280,281,286,290,293,301 , 305,314, 315,318,329,333,340,341,356,375,376,377,380,383,384,386,389,399,403,404,405,406 , 427,441,444,453,454,472,479,480.

In a preferred embodiment of the invention the variant comprise one or more of the following substitutions (using SEQ ID NO: 2 for the numbering):
G2P,A; M9I,L,F; H14Y; L15M,I,F,T; E16P; H26Y,Q,R,N; D27N,S,T; G48A,V,S,T; N49X; Q51X; A52X; D53E,Q,R; V54X; A58V,L,I,F; V73L,I,F; E84Q; G88X; D94X; N96Q; M103I,L,F; N104D; M/L107G,A,V,T,S; G108A; F111G,A,V,I,L,T; A114D,I,L,M,V,R; T125S; D128T,E; S130T,C; Y133F,H; W138F,Y; G140H,R,K,D,N; D142H,R,K,N; S144P; N148S; A149I; R156H,K,D,N; N161X; W165R; D166E; R168P; E171L,I,F; H173R,K,L; 1173L; L174I,F; A178N,Q,R,K,H; N179G, A,T,S; T180N,Q,R,K,H; N181X; N183X; W184R,K; D187N,S,T; E188P,T,I,S; N190F; D194X; L197X; G198X; S199X; N200X; 1201L,M,F,Y; D202X; F203L,I,F,M; S204X;H205X; E207Y,R; Q209V,L,I,F,M; E210X; E211Q; L212I,F; D214N, R,K,H; D221 N; E222Q,T; D224N,Q; Y228F; L230I,F; 1233A,V,L,F; K234N,Q; P237X; W239X; T241L,I,F,M; S242P,R; A252T; D253G,A,V,N; Q254K; D255N,Q,E,P; G260A; K264Q,S,T; D265N,Y; V267L,I,F,M; D275N,T; E276K; M277T,I, L,F; E280N,T,Q,S; M281H,I,L,F; V286X, preferably V286Y,L,I,F; Y290X; Y293H,F; S301G,A,D,K,E,R; R305A,K,Q,E, H,D,N; E314K,Q,R,S,T,H,N; A315K,R,S; 1318L,M,F; T329S; E333Q; A340R,K,N,D,Q,E; D341P,T,S,Q,N; G356Q,E,S, T,A; S375P; A376S; K377L,I,F,M; M380I,L,F; E383P,Q; L384I,F; D386N,Q,R,K,I,L; Q389K,R; Y399A,D,H; W403X; D404N; 1405L,F; V406I,L,F,A,D; N427X; H441K,N,D,Q,E; R442Q; Q444E,K,R; A445V; Q448A; H453R,K,Q,N; A454S, T,P; G472R, N479Q,K,R; Q480K,R.

In another preferred embodiment of the invention the variant comprise one or more of the following substitutions (using SEQ ID NO: 4 for the numbering):

G2P,A; M9I,L,F; H14Y; L15M,I,F,T; E16P; H26Y,Q,R,N; D27N,S,T; G48A,V,S,T; N49X; Q51X; A52X; D53E,Q,R; V54X; A58V,L,I,F; V73L,I,F; E84Q; G88X; D94X; N96Q; M103I,L,F; N104D; M/L107G,A,V,T,S,I,L,F; G108A; F111G,A,V,I,L, T; A114D,I,L,M,V,R; T125S; D128T,E; S130T,C; Y133F,H; W138F,Y; G140H,R,K,D,N; D142H,R,K,N; S144P; N148S; A149I; R156H,K,D,N; N161X; W165R; D166E; R168P; E171L,I,F; H173R,K,L; I173L; I174L,F; A178N,Q,R,K,H; N179G, A,T,S; T180N,Q,R,K,H; N181X; N183X; W184R,K; D187N,S,T; E188P,T,I,S; N190F; D194X; L197X; G198X; S199X; N200X; I201L,M,F,Y; D202X; F203L,I,F,M; S204X; H205X; E207Y,R; Q209V,L,I,F,M; D210X; D210E; E211Q; L212I,F; D214N,R,K,H; D221N; E222Q,T; D224N,Q; Y228F; L230I,F; I233A,V,L,F; K234N,Q; P237X; W239X; T241L,I,F,M; S242P,R; A252T; D253G,A,V,N; Q254K; D255N,Q,E,P; G260A; K264Q,S,T; D265N,Y; V267I,I,F,M; D275N,T; E276K; M277T,I,L,F; E280N,T,Q,S; M281H,I,L,F; V286X, preferably V286Y,L,I,F; Y290X; Y293H,F; S301G,A,D,K,E,R; R305A, K,Q,E,H,D,N; E314K,Q,R,S,T,H,N; A315K,R,S; M318L,I,F; T329S; E333,Q; A340R,K,N,D,Q,E; D341P,T,S,Q,N; G356Q,E,S,T,A; S375P; A376S; K377L,I,F,M; M380I,L,F; E383P,Q; L384I,F; D386N,Q,R,K,I,L; Q389K,R; Y399A,D, H; W403X; D404N; V405L,F,I; V406I,L,F,A,D; N427X; N441K,D,Q,E,H; R442Q; Q444E,K,R; A445V; Q448A; N453R, K,Q,H; G454A,S,T,P; G472R, N479Q,K,R; Q480K,R.

Within the scope of the invention are variants of other parent alpha-amylases (as defined herein) with one or more corresponding mutations.

In an embodiment of the invention a variant of the invention may comprise the following combination of substitutions:

> N49I+L/M107A;
> N49L+L/M107A;
> G48A+N49I+L/M107A;
> G48A+N49L+L/M107A;
> E188S,T,P+N190F+I201F+K264S;
> G48A+N49I+L/M107A+E188S,T,P+N190F+I201F+K264S;
> N190F+I201F;
> N190F+K264S;
> I201F+K264S;
> G140H+D142H+R156H,Y(+S144P);
> G140K+D142D+R156H,Y(+S144P);
> L197M+G198Y+S199A;
> L15T+E188S+Q209V+A376S+G472R;
> G48A+N49I+L/M107A+G140H+D142H+R156H,Y+E188P+N190F+I201F+K264S(+S144P);
> N49T+L/M107A+G140H+D142H+R156H,Y+E188P+N190F+I201 F+K264S(+S144P);

"/" before number indicate that KSM-K36 and KSM-K38 has different amino acid on the actual position. For instance L/M107A means that in KSM-K36 the mutation is L107A and in KSM-K38 the substitution is M107A.

_Altered pl_

Important positions and mutations with respect to achieving altered pl, in particular a higher pl, in particular at high pH (*i.e.,* pH 8-10.5) include any of the positions and mutations listed in the in "Altered Properties" section. It should be noted that when the alpha-amylase of the invention is for detergent use a high pl is desirable - for instance a pl in the range from 7-10.

_Stability_

Important positions and mutations with respect to achieving altered stability, in particular improved stability (i.e., higher or lower) at especially high pH (*i.e.,* pH 8-10.5) include any of the positions and mutations listed in the in "Altered Properties" section.

_Oxidation stability_

Variants of the invention may have altered oxidation stability, in particular higher oxidation stability, in comparison to the parent alpha-amylase.

In an embodiment such an alpha-amylase variant has one or more of Methionine amino acid residues exchanged with any amino acid residue except for Cys and Met. Thus, according to the invention the amino acid residues to replace the Methionine amino acid residue are the following: Ala, Arg, Asn, Asp, Gln, Glu, Gly, His, Ile, Leu, Lys, Phe, Pro, Ser, Thr, Trp, Tyr, and Val.

A preferred embodiment of the alpha-amylase of the invention is characterized by the fact that one or more of the Methionine amino acid residues is (are) exchanged with a Phe, Leu, Thr, Ala, Gly, Ser, Ile, or Val amino acid residue, preferably a Leu, Ile, Phe amino acid residue. In this embodiment a very satisfactory activity level and stability in the presence of oxidizing agents is obtained. Specifically this means that one or more of the Methionines in the following position may be replaced or deleted using any suitable technique known in the art, including especially site directed mutagenesis and gene shuffling. Target Methionine positions, using the SEQ ID NO: 2 numbering (KSM-K36), are one or more of M7, M8, M103, N277, M281, M304, M383, M428, M438.

Target Methionine positions, using the SEQ ID NO: 4 numbering (KSM-K38), are one or more of M7, M8, M103, M107, M197, M277, M281, M304, M318, M383, M428, M438.

In a preferred embodiment of the mutant alpha-amylase of the invention is characterized by the fact that the Methionine amino acid residue at position M107 and/or M277 and/or M281, and/or M318 and/or M383 and/or M428 is (are) exchanged with any of amino acid residue expect for Cys and Met, preferably with a Phe, Leu, Thr, Ala, Gly, Ser, Ile, or Asp. Also other parent alpha-amylases, as defined above, may have one or more Methionines substituted or deleted in particular in corresponding positions.

Specific activity

Important positions and mutations with respect to obtaining variants exhibiting altered specific activity, in particular increased or decreased specific activity, especially at temperatures from 10-60°C, preferably 20-50°C, especially 30-40°C, include any of the below positions and substitutions. The amino acid residues of particular importance are those involved in substrate binding. Primary target positions, using the SEQ ID NO: 2 numbering (KSM-K36), are one or more of G48, N49, Q51, A52, D53, V54, L107, G108, W165, D166, L197, G198, S199, K234, K264.

Primary target positions, using the SEQ ID NO: 4 numbering (KSM-K38), are one or more of G48, N49, Q51, A52, D53, V54, M107, G108, W165, D166, L197, G198, S199, K234, K264.

Preferred specific mutations/substitutions are the ones listed above in the section "Altered Properties" for the positions in question.

Altered pH profile

Important positions and mutations with respect to obtaining variants with altered pH profile, in particular improved activity at especially low pH (*i.e.,* pH 4-6) include mutations of amino residues located close to the active site residues, i.e., D229, E261, D328. Primary target positions, using the SEQ ID NO: 2 numbering (KSM-K36), are one or more of N104, E333 Primary target positions, using the SEQ ID NO: 4 numbering (KSM-K38), are one or more of N104, E333. Preferred specific mutations/substitutions are the ones listed above in the section "Altered properties" for the positions in question.

Altered alpha-amylase activity

A variant of the invention may have altered alpha-amylase activity, in particular increased alpha-amylase activity, in comparison to the parent alpha-amylase using the Phadebas® assay described below in the "Materials & Methods" section.

In a preferred embodiment of the invention an alpha-amylase substituted in a position corresponding to position 286 using the SEQ ID NO: 2 for the numbering has increased alpha-amylase activity. Preferred substitutions are V286Y, L,I,F.

In *Bacillus sp.* (SEQ ID NO: 2) the following substitution are result in increased activity: V286X (i.e., V286A,R, N,D,C,Q,E,G,H,I,L,K,M,F,P,S,T,W,Y), preferred are V286Y,L,I,F.

In *Bacillus sp.* (SEQ ID NO: 4) the following substitution are result in increased activity: A286X (i.e., V286R,N, D,C,Q,E,G,H,I,L,K,M,F,P,S,T,W,Y,V), preferred are A286Y,L,I,F.

Other mutations

Other preferred mutations to increase the stability of a particular protein include substitutions to a similar amino acid having a larger side chain, in order to fill out internal holds in the globular structure. Examples of these include glycine to alanine, alanine to valine, valine to isoleucine or leucine, alanine to serine, serine to threonine, asparagine to glutamine, aspartate to glutamate, phenyl to tyrosine or tryptophane, tyrosine to tryptophane, asparagine or asparatate to histidine, histidine to tyrosine and lysine to arginine substitutions, but are not limited to these examples only. Preferred mutations are Q84E, N96D, N121D, N393H, N444H.

Examples of larger mutations in SEQ 2 include: A315S/V and V101I. Examples of larger mutations in SEQ 4 include: V101I, A132V, D210E, A315S/V, V408I, S416T and A447V. Also other parent alpha-amylases, as defined above, may have one or more amino acid substituted into a larger amino acid, in particular in corresponding positions.

Other preferred mutations include substitutions of glycine residues to decrease the flexibility of the protein backbone. Examples of glycine substitutions in SEQ 2 and 4 includes: 19, 36, 48, 55, 57, 65, 71, 82, 88, 99, 108, 131, 140, 145, 162, 191, 198, 216, 227, 260, 268, 299, 300, 310, 332, 356, 357, 364, 368, 397, 410, 415, 423, 431, 433, 432, 441, 447, 454, 457, 464, 466, 468, 474, 475 and in particular G464A/S/N. Also other parent alpha-amylases, as defined above, may have one or more glycines substituted or deleted in particular in corresponding positions.

Other preferred mutations include introduction of proline residues in positions where possible with respect to limitations in the dihedral angles of the protein backbone and in the secondary structure. Examples of substitutions into proline in SEQ 2 include: W13, E16, Q51, L61, A109, G131, W182, D187, I233, I307, S334, W338, D341, W342, A379, S417. Examples of substitutions into proline in SEQ 4 include: W13, E16, Q51, L61, A109, G131, S144, W182, D187, 1233, 1307, S334, W338, D341, W342, A379, S417. Also other parent alpha-amylases, as defined above, may be stabilised by introduction of a proline, in particular in corresponding positions.

Important positions and mutations with respect to obtaining variants with improved stability at low pH are Asparagine substitutions. Preferred mutations include substitution or deletion of one or more Asparagine (Asn). Target Asparagines in SEQ ID NO: 2 (KSM-36) are N4, N17, N23, N34, N49, N68, N93, N96, N104, N121, N124, N147, N148, N161, N172, N179, N181, N183, N190, N192, N200, N278, N289, N291, N306, N326, N360, N371, N373, N393, N421, N430, N455, N463, N473, N482, which may be substituted with any other amino acid, or deleted, in particular N190F.

Target Aspargines in SEQ ID NO: 4 (KSM-38) are N17, N23, N49, N68, N93, N96, N104, N121, N124, N147, N148, N161, N172, N179, N181, N183, N190, N192, N200, N250, N278, N289, N291, N306, N326, N360, N371, N373, N393, 421, N430, N444, N455, N456, N463, N473, N482, which may be substituted with any other amino acid, or deleted, in particular N190F.

Also other parent alpha-amylases, as defined above, may have one or more Asparagines substituted or deleted in particular in corresponding positions.

Methods for preparing alpha-amylase variants of the invention

Several methods for introducing mutations into genes are known in the art. After a brief discussion of the cloning of alpha-amylase-encoding DNA sequences, methods for generating mutations at specific sites within the alpha-amylase-encoding sequence will be discussed.

Cloning a DNA sequence encoding an alpha-amylase

The DNA sequence encoding a parent alpha-amylase may be isolated from any cell or microorganism producing the alpha-amylase in question, using various methods well known in the art. First, a genomic DNA and/or cDNA library should be constructed using chromosomal DNA or messenger RNA from the organism that produces the alpha-amylase to be studied. Then, if the amino acid sequence of the alpha-amylase is known, homologous, labeled oligonucleotide probes may be synthesized and used to identify alpha-amylase-encoding clones from a genomic library prepared from the organism in question. Alternatively, a labeled oligonucleotide probe containing sequences homologous to a known alpha-amylase gene could be used as a probe to identify alpha-amylase-encoding clones, using hybridization and washing conditions of lower stringency.

Yet another method for identifying alpha-amylase-encoding clones would involve inserting fragments of genomic DNA into an expression vector, such as a plasmid, transforming alpha-amylase-negative bacteria with the resulting genomic DNA library, and then plating the transformed bacteria onto agar containing a substrate for alpha-amylase, thereby allowing clones expressing the alpha-amylase to be identified.

Alternatively, the DNA sequence encoding the enzyme may be prepared synthetically by established standard methods, e.g., the phosphoroamidite method described by S.L. Beaucage and M.H. Caruthers, Tetrahedron Letters 22, 1981, pp. 1859-1869, or the method described by Matthes et al., The EMBO J. 3, 1984, pp. 801-805. In the phosphoroamidite method, oligonucleotides are synthesized, e.g., in an automatic DNA synthesizer, purified, annealed, ligated and cloned in appropriate vectors.

Finally, the DNA sequence may be of mixed genomic and synthetic origin, mixed synthetic and cDNA origin or mixed genomic and cDNA origin, prepared by ligating fragments of synthetic, genomic or cDNA origin (as appropriate, the fragments corresponding to various parts of the entire DNA sequence), in accordance with standard techniques. The DNA sequence may also be prepared by polymerase chain reaction (PCR) using specific primers, for instance as described in US 4,683,202 or R.K. Saiki et al., Science 239, 1988, pp. 487-491.

Site-directed mutagenesis

Once an alpha-amylase-encoding DNA sequence has been isolated, and desirable sites for mutation identified, mutations may be introduced using synthetic oligonucleotides. These oligonucleotides contain nucleotide sequences flanking the desired mutation sites; mutant nucleotides are inserted during oligonucleotide synthesis. In a specific method, a single-stranded gap of DNA, bridging the alpha-amylase-encoding sequence, is created in a vector carrying the alpha-amylase gene. Then the synthetic nucleotide, bearing the desired mutation, is annealed to a homologous portion of the single-stranded DNA. The remaining gap is then filled in with DNA polymerase I (Klenow fragment) and the construct is ligated using T4 ligase. A specific example of this method is described in Morinaga et al., (1984, Biotechnology 2: 646-639). US patent no. 4,760,025 discloses the introduction of oligonucleotides encoding multiple mutations by performing minor alterations of the cassette. However, an even greater variety of mutations can be introduced at any one time by the Morinaga method, because a multitude of oligonucleotides, of various lengths, can be introduced.

Another method for introducing mutations into alpha-amylase-encoding DNA sequences is described in Nelson and Long, Analytical Biochemistry 180, 1989, pp. 147-151. It involves the 3-step generation of a PCR fragment containing the desired mutation introduced by using a chemically synthesized DNA strand as one of the primers in the PCR reactions. From the PCR-generated fragment, a DNA fragment carrying the mutation may be isolated by cleavage with restriction endonucleases and reinserted into an expression plasmid.

Random Mutagenesis

Random mutagenesis is suitably performed either as localised or region-specific random mutagenesis in at least three parts of the gene translating to the amino acid sequence shown in question, or within the whole gene.

The random mutagenesis of a DNA sequence encoding a parent alpha-amylase may be conveniently performed by use of any method known in the art.

In relation to the above, a further aspect of the present invention relates to a method for generating a variant of a parent alpha-amylase, e.g. wherein the variant exhibits altered or increased thermal stability relative to the parent, the method comprising:

(a) subjecting a DNA sequence encoding the parent alpha-amylase to random mutagenesis,
(b) expressing the mutated DNA sequence obtained in step (a) in a host cell, and
(c) screening for host cells expressing an alpha-amylase variant which has an altered property (e.g., pH-stability) relative to the parent alpha-amylase.

Step (a) of the above method of the invention is preferably performed using doped primers.

For instance, the random mutagenesis may be performed by use of a suitable physical or chemical mutagenizing agent, by use of a suitable oligonucleotide, or by subjecting the DNA sequence to PCR generated mutagenesis. Furthermore, the random mutagenesis may be performed by use of any combination of these mutagenizing agents. The mutagenizing agent may, e.g., be one that induces transitions, transversions, inversions, scrambling, deletions, and/or insertions.

Examples of a physical or chemical mutagenizing agent suitable for the present purpose include ultraviolet (UV) irradiation, hydroxylamine, N-methyl-N'-nitro-N-nitrosoguanidine (MNNG), O-methyl hydroxylamine, nitrous acid, ethyl methane sulphonate (EMS), sodium bisulphite, formic acid, and nucleotide analogues. When such agents are used, the mutagenesis is typically performed by incubating the DNA sequence encoding the parent enzyme to be mutagenized in the presence of the mutagenizing agent of choice under suitable conditions for the mutagenesis to take place, and selecting for mutated DNA having the desired properties.

When the mutagenesis is performed by the use of an oligonucleotide, the oligonucleotide may be doped or spiked with the three non-parent nucleotides during the synthesis of the oligonucleotide at the positions, which are to be changed. The doping or spiking may be done so that codons for unwanted amino acids are avoided. The doped or spiked oligonucleotide can be incorporated into the DNA encoding the alpha-amylase enzyme by any published technique, using e.g. PCR, LCR or any DNA polymerase and ligase as deemed appropriate.

Preferably, the doping is carried out using "constant random doping", in which the percentage of wild-type and mutation in each position is predefined. Furthermore, the doping may be directed toward a preference for the introduction of certain nucleotides, and thereby a preference for the introduction of one or more specific amino acid residues. The doping may be made, e.g., so as to allow for the introduction of 90% wild type and 10% mutations in each position. An additional consideration in the choice of a doping scheme is based on genetic as well as protein-structural constraints. The doping scheme may be made using the DOPE program (see "Material and Methods" section), which, *inter alia,* ensures that introduction of stop codons is avoided.

When PCR-generated mutagenesis is used, either a chemically treated or non-treated gene encoding a parent alpha-amylase is subjected to PCR under conditions that increase the mis-incorporation of nucleotides (Deshler 1992; Leung et al., Technique, Vol.1, 1989, pp. 11-15).

A mutator strain of *E. coli* (Fowler et al., Molec. Gen. Genet., 133, 1974, pp. 179-191), S. *cereviseae* or any other microbial organism may be used for the random mutagenesis of the DNA encoding the alpha-amylase by, e.g., transforming a plasmid containing the parent glycosylase into the mutator strain, growing the mutator strain with the plasmid and isolating the mutated plasmid from the mutator strain. The mutated plasmid may be subsequently transformed into the expression organism.

The DNA sequence to be mutagenized may be conveniently present in a genomic or cDNA library prepared from an organism expressing the parent alpha-amylase. Alternatively, the DNA sequence may be present on a suitable vector such as a plasmid or a bacteriophage, which as such may be incubated with or otherwise exposed to the mutagenising agent. The DNA to be mutagenized may also be present in a host cell either by being integrated in the genome of said cell or by being present on a vector harboured in the cell. Finally, the DNA to be mutagenized may be in isolated form. It will be understood that the DNA sequence to be subjected to random mutagenesis is preferably a cDNA or a genomic DNA sequence.

In some cases it may be convenient to amplify the mutated DNA sequence prior to performing the expression step b) or the screening step c). Such amplification may be performed in accordance with methods known in the art, the presently preferred method being PCR-generated amplification using oligonucleotide primers prepared on the basis of the DNA or amino acid sequence of the parent enzyme.

Subsequent to the incubation with or exposure to the mutagenising agent, the mutated DNA is expressed by culturing a suitable host cell carrying the DNA sequence under conditions allowing expression to take place. The host cell used for this purpose may be one which has been transformed with the mutated DNA sequence, optionally present on a vector, or one which was carried the DNA sequence encoding the parent enzyme during the mutagenesis treatment. Examples of suitable host cells are the following: gram positive bacteria such as *Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, Bacillus lautus, Bacillus megaterium, Bacillus thuringiensis, Streptomyces lividans* or *Streptomyces murinus;* and gram-negative bacteria such as *E. coli* or *Pseudomonas.*

The mutated DNA sequence may further comprise a DNA sequence encoding functions permitting expression of the mutated DNA sequence.

Localized random mutagenesis

The random mutagenesis may be advantageously localized to a part of the parent alpha-amylase in question. This may, e.g., be advantageous when certain regions of the enzyme have been identified to be of particular importance for a given property of the enzyme, and when modified are expected to result in a variant having improved properties. Such regions may normally be identified when the tertiary structure of the parent enzyme has been elucidated and related to the function of the enzyme.

The localized, or region-specific, random mutagenesis is conveniently performed by use of PCR generated mutagenesis techniques as described above or any other suitable technique known in the art. Alternatively, the DNA sequence encoding the part of the DNA sequence to be modified may be isolated, e.g., by insertion into a suitable vector, and said part may be subsequently subjected to mutagenesis by use of any of the mutagenesis methods discussed above.

Alternative methods of providing alpha-amylase variants

Alternative methods for providing variants of the invention include gene-shuffling method known in the art including the methods, e.g., described in WO 95/22625 (from Affymax Technologies N.V.) and WO 96/00343 (from Novo Nordisk A/S).

Expression of alpha-amylase variants

Expresion Vectors

According to the invention, a DNA sequence encoding the variant produced by methods described above, or by any alternative methods known in the art, can be expressed, in enzyme form, using an expression vector which typically includes control sequences encoding a promoter, operator, ribosome binding site, translation initiation signal, and, optionally, a repressor gene or various activator genes.

The recombinant expression vector carrying the DNA sequence encoding an alpha-amylase variant of the invention may be any vector, which may conveniently be subjected to recombinant DNA procedures, and the choice of vector will often depend on the host cell into which it is to be introduced. Thus, the vector may be an autonomously replicating vector, i.e., a vector which exists as an extrachromosomal entity, the replication of which is independent of chromosomal replication, e.g., a plasmid, a bacteriophage or an extrachromosomal element, minichromosome or an artificial chromosome. Alternatively, the vector may be one which, when introduced into a host cell, is integrated into the host cell genome and replicated together with the chromosome(s) into which it has been integrated.

Promoters

In the vector, the DNA sequence should be operably connected to a suitable promoter sequence. The promoter may be any DNA sequence, which shows transcriptional activity in the host cell of choice and may be derived from genes encoding proteins either homologous or heterologous to the host cell. Examples of suitable promoters for directing the transcription of the DNA sequence encoding an alpha-amylase variant of the invention, especially in a bacterial host, are the promoter of the lac operon of *E.coli,* the *Streptomyces coelicolor* agarase gene *dag*A promoters, the promoters of the *Bacillus licheniformis* alpha-amylase gene *(amyL),* the promoters of the *Bacillus stearothermophilus* maltogenic amylase gene (*amyM*), the promoters of the *Bacillus amyloliquefaciens* alpha-amylase *(amyQ),* the promoters of the *Bacillus subtilis* xylA and xylB genes etc. For transcription in a fungal host, examples of useful promoters are those derived from the gene encoding *A. oryzae* TAKA amylase, *Rhizomucor miehei* aspartic proteinase, *A. niger* neutral alpha-amylase, *A. niger* acid stable alpha-amylase, *A. niger* glucoamylase, *Rhizomucor miehei* lipase, *A. oryzae* alkaline protease, *A. oryzae* triose phosphate isomerase or *A. nidulans* acetamidase.

Transcription terminators

The expression vector of the invention may also comprise a suitable transcription terminator and, in eukaryotes, polyadenylation sequences operably connected to the DNA sequence encoding the alpha-amylase variant of the invention. Termination and polyadenylation sequences may suitably be derived from the same sources as the promoter.

Replication sequences

The vector may further comprise a DNA sequence enabling the vector to replicate in the host cell in question. Examples of such sequences are the origins of replication of plasmids pUC19, pACYC177, pUB110, pE194, pAMB1 and pIJ702.

Selectable markers

The vector may also comprise a selectable marker, e.g., a gene the product of which complements a defect in the host cell, such as the *dal* genes from *B. subtilis* or *B. licheniformis,* or one which confers antibiotic resistance such as ampicillin, kanamycin, chloramphenicol or tetracyclin resistance. Furthermore, the vector may comprise *Aspergillus* selection markers such as amdS, argB, niaD and sC, a marker giving rise to hygromycin resistance, or the selection may be accomplished by co-transformation, e.g., as described in WO 91/17243.

Secretion sequences

While intracellular expression may be advantageous in some respects, e.g., when using certain bacteria as host cells, it is generally preferred that the expression is extracellular. In general, the *Bacillus* alpha-amylases mentioned herein comprise a preregion permitting secretion of the expressed protease into the culture medium. If desirable, this preregion may be replaced by a different preregion or signal sequence, conveniently accomplished by substitution of the DNA sequences encoding the respective preregions.

Host cells

The procedures used to ligate the DNA construct of the invention encoding an alpha-amylase variant, the promoter, terminator and other elements, respectively, and to insert them into suitable vectors containing the information necessary for replication, are well known to persons skilled in the art (cf., for instance, Sambrook et al., Molecular Cloning: A Laboratory Manual, 2nd Ed., Cold Spring Harbor, 1989).

The cell of the invention, either comprising a DNA construct or an expression vector of the invention as defined above, is advantageously used as a host cell in the recombinant production of an alpha-amylase variant of the invention. The cell may be transformed with the DNA construct of the invention encoding the variant, conveniently by integrating the DNA construct (in one or more copies) in the host chromosome. This integration is generally considered to be an advantage as the DNA sequence is more likely to be stably maintained in the cell. Integration of the DNA constructs into the host chromosome may be performed according to conventional methods, e.g., by homologous or heterologous recombination. Alternatively, the cell may be transformed with an expression vector as described above in connection with the different types of host cells.

The cell of the invention may be a cell of a higher organism such as a mammal or an insect, but is preferably a microbial cell, e.g., a bacterial or a fungal (including yeast) cell.

Examples of suitable bacteria are Gram-positive bacteria such as *Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, Bacillus lautus, Bacillus megaterium, Bacillus thuringiensis,* or *Streptomyces lividans* or *Streptomyces murinus,* or gramne-gative bacteria such as *E.coli or pseudomonas.* The transformation of the bacteria may, for instance, be effected by protoplast transformation or by using competent cells in a manner known *per se.*

The yeast organism may favorably be selected from a species of *Saccharomyces* or *Schizosaccharomyces,* e.g. *Saccharomyces cerevisiae.* The filamentous fungus may advantageously belong to a species of *Aspergillus,* e.g., *Aspergillus oryzae* or *Aspergillus niger.* Fungal cells may be transformed by a process involving protoplast formation and transformation of the protoplasts followed by regeneration of the cell wall in a manner known *per se.* A suitable procedure for transformation of *Aspergillus* host cells is described in EP 238 023.

Method of producing an alpha-amylase variant of the invention

In a yet further aspect, the present invention relates to a method of producing an alpha-amylase variant of the invention, which method comprises cultivating a host cell as described above under conditions conducive to the production of the variant and recovering the variant from the cells and/or culture medium.

The medium used to cultivate the cells may be any conventional medium suitable for growing the host cell in question and obtaining expression of the alpha-amylase variant of the invention. Suitable media are available from commercial suppliers or may be prepared according to published recipes (e.g., as described in catalogues of the American Type Culture Collection).

The alpha-amylase variant secreted from the host cells may conveniently be recovered from the culture medium by well-known procedures, including separating the cells from the medium by centrifugation or filtration, and precipitating proteinaceous components of the medium by means of a salt such as ammonium sulphate, followed by the use of chromatographic procedures such as ion exchange chromatography, affinity chromatography, or the like.

Industrial Applications

Owing to their activity at alkaline pH values, the alpha-amylase variants of the invention are well suited for use in a variety of industrial processes, in particular the enzyme finds potential applications as a component in detergents, e.g., laundry, dishwashing and hard surface cleaning detergent compositions, but it may also be useful for desizing of textiles, fabrics and garments, beer making or brewing, in pulp and paper production, and further in the production of sweeteners and ethanol (see for instance US patent no. 5,231,017 - hereby incorporated by reference), such as fuel, drinking and industrial ethanol, from starch or whole grains.

Starch Conversion

Conventional starch-conversion processes, such as liquefaction and saccharification processes are described, e.g., in US Patent No. 3,912,590 and EP patent publications Nos. 252,730 and 63,909, hereby incorporated by reference.

A "traditional" starch conversion process degrading starch to lower molecular weight carbohydrate components such as sugars or fat replacers includes a debranching step.

Starch to sugar conversion

In the case of converting starch into a sugar the starch is depolymerized. A such depolymerization process consists of a Pre-treatment step and two or three consecutive process steps, *viz.* a liquefaction process, a saccharification process and dependent on the desired end product optionally an isomerization process.

Pre-treatment of native starch

Native starch consists of microscopic granules, which are insoluble in water at room temperature. When an aqueous starch slurry is heated, the granules swell and eventually burst, dispersing the starch molecules into the solution. During this "gelatinization" process there is a dramatic increase in viscosity. As the solids level is 30-40% in a typically industrial process, the starch has to be thinned or "liquefied" so that it can be handled. This reduction in viscosity is today mostly obtained by enzymatic degradation.

Liquefaction

During the liquefaction step, the long chained starch is degraded into branched and linear shorter units (maltodextrins)
by an alpha-amylase. The liquefaction process is carried out at 105-110°C for 5 to 10 minutes followed by 1-2 hours at 95°C. The pH lies between 5.5 and 6.2. In order to ensure optimal enzyme stability under these conditions, 1 mM of calcium is added (40 ppm free calcium ions). After this treatment the liquefied starch will have a "dextrose equivalent" (DE) of 10-15.

Saccharification

After the liquefaction process the maltodextrins are converted into dextrose by addition of a glucoamylase (e.g., AMG™) and a debranching enzyme, such as an isoamylase (US Patent 4,335,208) or a pullulanase (e.g., Promozyme™) (US Patent 4,560,651). Before this step the pH is reduced to a value below 4.5, maintaining the high temperature (above 95°C) to inactivate the liquefying alpha-amylase to reduce the formation of short oligosaccharide called "panose precursors" which cannot be hydrolyzed properly by the debranching enzyme.
The temperature is lowered to 60°C, and glucoamylase and debranching enzyme are added. The saccharification process proceeds for 24-72 hours.
Normally, when denaturing the $\alpha$-amylase after the liquefaction step about 0.2-0.5% of the saccharification product is the branched trisaccharide $6^2$-alpha-glucosyl maltose (panose) which cannot be degraded by a pullulanase. If active amylase from the liquefaction step is present during saccharification (i.e., no denaturing), this level can be as high as 1-2%, which is highly undesirable as it lowers the saccharification yield significantly.

Isomerization

When the desired final sugar product is, e.g., high fructose syrup the dextrose syrup may be converted into fructose. After the saccharification process the pH is increased to a value in the range of 6-8, preferably pH 7.5, and the calcium is removed by ion exchange. The dextrose syrup is then converted into high fructose syrup using, e.g., an immmobilized glucoseisomerase (such as Sweetzyme™ IT).

Ethanol production

In general alcohol production (ethanol) from whole grain can be separated into 4 main steps

- Milling
- Liquefaction
- Saccharification
- Fermentation

Milling

The grain is milled in order to open up the structure and allowing for further processing. Two processes are used wet or dry milling. In dry milling the whole kernel is milled and used in the remaining part of the process. Wet milling gives a very good separation of germ and meal (starch granules and protein) and is with a few exceptions applied at locations where there is a parallel production of syrups.

Liquefaction

In the liquefaction process the starch granules are solubilized by hydrolysis to maltodextrins mostly of a DP higher than 4. The hydrolysis may be carried out by acid treatment or enzymatically by alpha-amylase. Acid hydrolysis is used on a limited basis. The raw material can be milled whole grain or a side stream from starch processing.
Enzymatic liquefaction is typically carried out as a three-step hot slurry process. The slurry is heated to between 60-95 C, preferably 80-85 C, and the enzyme(s) is (are) added. Then the slurry is jet-cooked at between 95-140 C, preferably 105-125 C, cooled to 60-95 C and more enzyme(s) is (are) added to obtain the final hydrolysis. The liquefaction process is carried out at pH 4.5-6.5, typically at a pH between 5 and 6. Milled and liquefied grain is also known as mash.

Saccharification

To produce low molecular sugars $DP_{1-3}$ that can be metabolized by yeast, the maltodextrin from the liquefaction must be further hydrolyzed. The hydrolysis is typically done enzymatically by glucoamylases, alternatively alpha-glucosidases or acid alpha-amylases can be used. A full saccharification step may last up to 72 hours, however, it is common only to do a pre-saccharification of typically 40-90 minutes and then complete saccharification during fermentation (SSF). Saccharification is typically carried out at temperatures from 30-65 C, typically around 60 C, and at pH 4.5.

Fermentation

Yeast typically from *Saccharomyces* spp. is added to the mash and the fermentation is ongoing for 24-96 hours, such as typically 35-60 hours. The temperature is between 26-34 C, typically at about 32 C, and the pH is from pH 3-6, preferably around pH 4-5.
Note that the most widely used process is a simultaneous saccharification and fermentation (SSF) process where there is no holding stage for the saccharification, meaning that yeast and enzyme is added together. When doing SSF it is common to introduce a pre-saccharification step at a temperature above 50 C, just prior to the fermentation.

Distillation

Following the fermentation the mash is distilled to extract the ethanol.
The ethanol obtained according to the process of the invention may be used as, e.g., fuel ethanol; drinking ethanol, i.e., potable neutral spirits; or industrial ethanol.

By-products

Left over from the fermentation is the grain, which is typically used for animal feed either in liquid form or dried.
Further details on how to carry out liquefaction, saccharification, fermentation, distillation, and recovering of ethanol are well known to the skilled person.
According to the process of the invention the saccharification and fermentation may be carried out simultaneously or separately.

Pulp and Paper Production

The alkaline alpha-amylase of the invention may also be used in the production of lignocellulosic materials, such as pulp, paper and cardboard, from starch reinforced waste paper and cardboard, especially where re-pulping occurs at pH above 7 and where amylases facilitate the disintegration of the waste material through degradation of the reinforcing starch. The alpha-amylase of the invention is especially useful in a process for producing a papermaking pulp from starch-coated printed-paper. The process may be performed as described in WO 95/14807, comprising the following steps:

a) disintegrating the paper to produce a pulp,
b) treating with a starch-degrading enzyme before, during or after step a), and
c) separating ink particles from the pulp after steps a) and b).

The alpha-amylases of the invention may also be very useful in modifying starch where enzymatically modified starch is used in papermaking together with alkaline fillers such as calcium carbonate, kaolin and clays. With the alkaline alpha-amylases of the invention it becomes possible to modify the starch in the presence of the filler thus allowing for a simpler integrated process.

Desizing of Textiles, Fabrics and Garments

An alpha-amylase of the invention may also be very useful in textile, fabric or garment desizing. In the textile processing industry, alpha-amylases are traditionally used as auxiliaries in the desizing process to facilitate the removal of starch-containing size, which has served as a protective coating on weft yarns during weaving. Complete removal of the size coating after weaving is important to ensure optimum results in the subsequent processes, in which the fabric is scoured, bleached and dyed. Enzymatic starch breakdown is preferred because it does not involve any harmful effect on the fiber material. In order to reduce processing cost and increase mill throughput, the desizing processing is sometimes combined with the scouring and bleaching steps. In such cases, non-enzymatic auxiliaries such as alkali or oxidation agents are typically used to break down the starch, because traditional alpha-amylases are not very compatible with high pH levels and bleaching agents. The non-enzymatic breakdown of the starch size does lead to some fiber damage because of the rather aggressive chemicals used. Accordingly, it would be desirable to use the alpha-amylases of the invention as they have an improved performance in alkaline solutions. The alpha-amylases may be used alone or in combination with a cellulase when desizing cellulose-containing fabric or textile.

Desizing and bleaching processes are well known in the art. For instance, such processes are described in WO 95/21247, US patent 4,643,736, EP 119,920 hereby in corporate by reference.

Commercially available products for desizing include Aquazyme® and Aquazyme® Ultra from Novo Nordisk A/S.

Beer making

The alpha-amylases of the invention may also be very useful in a beer-making process; the alpha-amylases will typically be added during the mashing process.

Detergent Compositions

The alpha-amylase of the invention may be added to and thus become a component of a detergent composition.

The detergent composition of the invention may for example be formulated as a hand or machine laundry detergent composition including a laundry additive composition suitable for pre-treatment of stained fabrics and a rinse added fabric softener composition, or be formulated as a detergent composition for use in general household hard surface cleaning operations, or be formulated for hand or machine dishwashing operations.

In a specific aspect, the invention provides a detergent additive comprising the enzyme of the invention. The detergent additive as well as the detergent composition may comprise one or more other enzymes such as a protease, a lipase, a cutinase, an amylase, a carbohydrase, a cellulase, a pectinase, a mannanase, an arabinase, a galactanase, a xylanase, an oxidase, e.g., a laccase, a pectate lyase, and/or a peroxidase.

In general the properties of the chosen enzyme(s) should be compatible with the selected detergent, (i.e., pH-optimum, compatibility with other enzymatic and non-enzymatic ingredients, etc.), and the enzyme(s) should be present in effective amounts.

Proteases: Suitable proteases include those of animal, vegetable or microbial origin. Microbial origin is preferred. Chemically modified or protein engineered mutants are included. The protease may be a serine protease or a metallo protease, preferably an alkaline microbial protease or a trypsin-like protease. Examples of alkaline proteases are subtilisins, especially those derived from Bacillus, e.g., subtilisin Novo, subtilisin Carlsberg, subtilisin 309, subtilisin 147 and subtilisin 168 (described in WO 89/06279). Examples of trypsin-like pro-teases are trypsin (e.g., of porcine or bovine origin) and the Fusarium protease described in WO 89/06270 and WO 94/25583.

Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235 and 274.

Preferred commercially available protease enzymes include Alcalase®, Savinase®, Primase®, Duralase®, Esperase®, and Kannase® (Novo Nordisk A/S), Maxatase®, Maxacal, Maxapem®, Properase®, Purafect®, Purafect OxP®, FN2®, and FN3® (Genencor International Inc.).

Lipases: Suitable lipases include those of bacterial or fungal origin. Chemically modified or protein engineered mutants are included. Examples of useful lipases include lipases from *Humicola* (synonym *Thermomyces),* e.g., from *H. lanuginosa (T. lanuginosus)* as described in EP 258 068 and EP 305 216 or from *H. insolens* as described in WO 96/13580, a Pseudomonas lipase, e.g., from *P. alcaligenes* or *P. pseudoalcaligenes* (EP 218 272), *P. cepacia* (EP 331 376), *P. stutzeri* (GB 1,372,034), *P. fluorescens, Pseudomonas* sp. strain SD 705 (WO 95/06720 and WO 96/27002), *P. wisconsinensis* (WO 96/12012), a *Bacillus* lipase, e.g., from *B. subtilis* (Dartois et al. (1993), Biochemica et Biophysica Acta, 1131, 253-360), *B. stearothermophilus* (JP 64/744992) or *B. pumilus* (WO 91/16422). Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202.

Preferred commercially available lipase enzymes include Lipolase™ and Lipolase Ultra™ (Novo Nordisk A/S).

Amylases: Suitable amylases (alpha and/or beta) include those of bacterial or fungal origin. Chemically modified or protein engineered mutants are included. Amylases include, for example, alpha-amylases obtained from *Bacillus,* e.g., a special strain of *B. licheniformis,* described in more detail in GB 1,296,839. Examples of useful alpha-amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are Duramyl™, Termamyl™, Natalase™, Fungamyl™ and BAN™ (Novo Nordisk A/S), Rapidase™ and Purastar™ (from Genencor International Inc.).

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* e.g., the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellu-lases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme®, and Carezyme® (Novo Nordisk A/S), Clazinase®, and Puradax HA® (Genencor International Inc.), and KAC-500(B)® (Kao Corporation).

Peroxidases/Oxidases: Suitable peroxidases/oxidases include those of plant, bacterial or fungal origin. Chemically modified or protein engineered mutants are included. Examples of useful peroxidases include peroxidases from Coprinus, e.g., from *C. cinereus,* and variants thereof as those described in WO 93/24618, WO 95/10602, and WO 98/15257.

Commercially available peroxidases include Guardzyme® (Novo Nordisk A/S).

Pectate lyase: Many pectate lyases have been described in the art, see e.g. WO 99/27083 (Novozymes A/S) or WO 99/27084 (Novozymes A/S), both of which are incorporated herein by reference in their totality.

The detergent enzyme(s) may be included in a detergent composition by adding separate additives containing one or more enzymes, or by adding a combined additive comprising all of these enzymes. A detergent additive of the invention, i.e., a separate additive or a combined additive, can be formulated, e.g., granulate, a liquid, a slurry, etc. Preferred detergent additive formulations are granulates, in particular non-dusting granulates, liquids, in particular stabilized liquids, or slurries.

Non-dusting granulates may be produced, e.g., as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonyl-phenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme pre-parations may, for instance, be stabilized by adding a polyol such as propylene glycol, a sugar or sugar alcohol, lactic acid or boric acid according to established methods. Protected enzymes may be prepared according to the method disclosed in EP 238,216.

The detergent composition of the invention may be in any convenient form, e.g., a bar, a tablet, a powder, a granule, a paste or a liquid. A liquid detergent may be aqueous, typically containing up to 70 % water and 0-30 % organic solvent, or non-aqueous.

The detergent composition comprises one or more surfactants, which may be non-ionic including semi-polar and/or anionic and/or cationic and/or zwitterionic. The surfactants are typically present at a level of from 0.1% to 60% by weight.

When included therein the detergent will usually contain from about 1% to about 40% of an anionic surfactant such as linear alkylbenzenesulfonate, alpha-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid or soap.

When included therein the detergent will usually contain from about 0.2% to about 40% of a non-ionic surfactant such as alcohol ethoxylate, nonyl-phenol ethoxylate, alkylpolyglycoside, alkyldimethylamine-oxide, ethoxylated fatty acid monoethanol-amide, fatty acid monoethanolamide, polyhydroxy alkyl fatty acid amide, or N-acyl N-alkyl derivatives of glucosamine ("glucamides").

The detergent may contain 0-65 % of a detergent builder or complexing agent such as zeolite, diphosphate, tripho-sphate, phosphonate, carbonate, citrate, nitrilotriacetic acid, ethylenediaminetetraacetic acid, diethylenetri-aminepen-taacetic acid, alkyl- or alkenylsuccinic acid, soluble silicates or layered silicates (e.g. SKS-6 from Hoechst).

The detergent may comprise one or more polymers. Examples are carboxymethylcellulose, poly(vinyl-pyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid co-polymers.

The detergent may contain a bleaching system, which may comprise a $H_2O_2$ source such as perborate or percarbonate which may be combined with a peracid-forming bleach activator such as tetraacetylethylenediamine or nonanoyloxyben-zenesul-fonate. Alternatively, the bleaching system may comprise peroxyacids of, e.g., the amide, imide, or sulfone type.

The enzyme(s) of the detergent composition of the inven-tion may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the com-position may be formulated as described in, e.g., WO 92/19709 and WO 92/19708.

The detergent may also contain other conventional detergent ingredients such as e.g. fabric conditioners including clays, foam boosters, suds suppressors, anti-corrosion agents, soil-suspending agents, anti-soil re-deposition agents, dyes, bactericides, optical brighteners, hydrotropes, tarnish inhibitors, or perfumes.

It is at present contemplated that in the detergent compositions any enzyme, in particular the enzyme of the invention, may be added in an amount corresponding to 0.01-100 mg of enzyme protein per liter of wash liquor, preferably 0.05-5 mg of enzyme protein per liter of wash liquor, in particular 0.1-1 mg of enzyme protein per liter of wash liquor.

The enzyme of the invention may additionally be incorporated in the detergent formulations disclosed in WO 97/07202, which is hereby incorporated as reference.

Dishwash Deterget Compositions

The enzyme of the invention mat also be used in dish wash detergent compositions, including the following:

1) POWDERAUTOMATIC DISHWASHING COMPOSITION

| Nonionic surfactant | 0.4 - 2.5% |
|---|---|
| Sodium metasilicate | 0 - 20% |
| Sodium disilicate | 3 - 20% |
| Sodium triphosphate | 20 - 40% |
| Sodium carbonate | 0 - 20% |
| Sodium perborate | 2 - 9% |
| Tetraacetyl ethylene diamine (TAED) | 1 - 4% |
| Sodium sulphate | 5 - 33% |
| Enzymes | 0.0001 - 0.1% |

2) POWDER AUTOMATIC DISHWASHING COMPOSITION

| Nonionic surfactant (e.g. alcohol ethoxylate) | 1 - 2% |
|---|---|
| Sodium disilicate | 2 - 30% |
| Sodium carbonate | 10 -50% |
| Sodium phosphonate | 0 - 5% |
| Trisodium citrate dihydrate | 9 - 30% |
| Nitrilotrisodium acetate (NTA) | 0 - 20% |
| Sodium perborate monohydrate | 5 -10% |
| Tetraacetyl ethylene diamine (TAED) | 1 - 2% |
| Polyacrylate polymer (e.g. maleic acid/acrylic acid copolymer) | 6 - 25% |
| Enzymes | 0.0001 - 0.1% |
| Perfume | 0.1 - 0.5% |
| Water | 5 - 10 |

3) POWDER AUTOMATIC DISHWASHING COMPOSITION

| Nonionic surfactant | 0.5 - 2.0% |
|---|---|
| Sodium disilicate | 25 - 40% |
| Sodium citrate | 30 - 55% |
| Sodium carbonate | 0 - 29% |

(continued)

| Sodium bicarbonate | 0 - 20% |
|---|---|
| Sodium perborate monohydrate | 0 - 15% |
| Tetraacetyl ethylene diamine (TAED) | 0 - 6% |
| Maleic acid/acrylic acid copolymer | 0 - 5% |
| Clay | 1 - 3% |
| Polyamino acids | 0 - 20% |
| Sodium polyacrylate | 0 - 8% |
| Enzymes | 0.0001 - 0.1% |

4) POWDER AUTOMATIC DISHWASHING COMPOSITION

| Nonionic surfactant | 1 - 2% |
|---|---|
| Zeolite MAP | 15 - 42% |
| Sodium disilicate | 30 - 34% |
| Sodium citrate | 0 - 12% |
| Sodium carbonate | 0 - 20% |
| Sodium perborate monohydrate | 7 - 15% |
| Tetraacetyl ethylene diamine (TAED) | 0 - 3% |
| Polymer | 0 - 4% |
| Maleic acid/acrylic acid copolymer | 0 - 5% |
| Organic phosphonate | 0 - 4% |
| Clay | 1 - 2% |
| Enzymes | 0.0001 - 0.1% |
| Sodium sulphate | Balance |

5) POWDER AUTOMATIC DISHWASHING COMPOSITION

| Nonionic surfactant | 1 - 7% |
|---|---|
| Sodium disilicate | 18 - 30% |
| Trisodium citrate | 10 - 24% |
| Sodium carbonate | 12 - 20% |
| Monopersulphate (2 $KHSO_5.KHSO_4.K_2SO_4$) | 15 - 21 % |
| Bleach stabilizer | 0.1 - 2% |
| Maleic acid/acrylic acid copolymer | 0 - 6% |
| Diethylene triamine pentaacetate, pentasodium salt | 0 - 2.5% |
| Enzymes | 0.0001 - 0.1% |
| Sodium sulphate, water | Balance |

6) POWDER AND LIQUID DISHWASHING COMPOSITION WITH CLEANING SURFACTANT SYSTEM

| Nonionic surfactant | 0 - 1.5% |
|---|---|

(continued)

| Octadecyl dimethylamine N-oxide dihydrate | 0 - 5% |
|---|---|
| 80:20 wt.C18/C16 blend of octadecyl dimethylamine N-oxide dihydrate and hexadecyldimethyl amine N-oxide dihydrate | 0 - 4% |
| 70:30 wt.C18/C16 blend of octadecyl bis (hydroxyethyl)amine N-oxide anhydrous and hexadecyl bis (hydroxyethyl)amine N-oxide anhydrous | 0 - 5% |
| $C_{13}$-$C_{15}$ alkyl ethoxysulfate with an average degree of ethoxylation of 3 | 0 - 10% |
| $C_{12}$-$C_{15}$ alkyl ethoxysulfate with an average degree of ethoxylation of 3 | 0 - 5% |
| $C_{13}$-$C_{15}$ ethoxylated alcohol with an average degree of ethoxylation of 12 | 0 - 5% |
| A blend of $C_{12}$-$C_{15}$ ethoxylated alcohols with an average degree of ethoxylation of 9 | 0 - 6.5% |
| A blend of $C_{13}$-$C_{15}$ ethoxylated alcohols with an average degree of ethoxylation of 30 | 0 - 4% |
| Sodium disilicate | 0 - 33% |
| Sodium tripolyphosphate | 0 - 46% |
| Sodium citrate | 0 - 28% |
| Citric acid | 0 - 29% |
| Sodium carbonate | 0 - 20% |
| Sodium perborate monohydrate | 0 - 11.5% |
| Tetraacetyl ethylene diamine (TAED) | 0 - 4% |
| Maleic acid/acrylic acid copolymer | 0 - 7.5% |
| Sodium sulphate | 0 - 12.5% |
| Enzymes | 0.0001 - 0.1% |

7) NON-AQUEOUS LIQUID AUTOMATIC DISHWASHING COMPOSITION

| Liquid nonionic surfactant (e.g. alcohol ethoxylates) | 2.0 -10.0% |
|---|---|
| Alkali metal silicate | 3.0 -15.0% |
| Alkali metal phosphate | 20.0 - 40.0% |
| Liquid carrier selected from higher glycols, polyglycols, polyoxides, glycolethers | 25.0 - 45.0% |
| Stabilizer (e.g. a partial ester of phosphoric acid and a $C_{16}$-$C_{18}$ alkanol) | 0.5 - 7.0% |
| Foam suppressor (e.g. silicone) | 0 - 1.5% |
| Enzymes | 0.0001 - 0.1% |

8) NON-AQUEOUS LIQUID DISHWASHING COMPOSITION

| Liquid nonionic surfactant (e.g. alcohol ethoxylates) | 2.0 - 10.0% |
|---|---|
| Sodium silicate | 3.0 - 15.0% |
| Alkali metal carbonate | 7.0 - 20.0% |
| Sodium citrate | 0.0 - 1.5% |
| Stabilizing system (e.g. mixtures of finely divided silicone and low molecular weight dialkyl polyglycol ethers) | 0.5 - 7.0% |
| Low molecule weight polyacrylate polymer | 5.0 - 15.0% |
| Clay gel thickener (e.g. bentonite) | 0.0 - 10.0% |

(continued)

| Hydroxypropyl cellulose polymer | 0.0 - 0.6% |
|---|---|
| Enzymes | 0.0001 - 0.1% |
| Liquid carrier selected from higher lycols, polyglycols, polyoxides and glycol ethers | Balance |

9) THIXOTROPIC LIQUID AUTOMATIC DISHWASHING COMPOSITION

| $C_{12}$-$C_{14}$ fatty acid | 0 - 0.5% |
|---|---|
| Block co-polymer surfactant | 1.5 - 15.0% |
| Sodium citrate | 0 - 12% |
| Sodium tripolyphosphate | 0 - 15% |
| Sodium carbonate | 0 - 8% |
| Aluminium tristearate | 0 - 0.1% |
| Sodium cumene sulphonate | 0 - 1.7% |
| Polyacrylate thickener | 1.32 - 2.5% |
| Sodium polyacrylate | 2.4 - 6.0% |
| Boric acid | 0 - 4.0% |
| Sodium formate | 0 - 0.45% |
| Calcium formate | 0 - 0.2% |

| Sodium n-decydiphenyl oxide disulphonate | 0 - 4.0% |
|---|---|
| Monoethanol amine (MEA) | 0 - 1.86% |
| Sodium hydroxide (50%) | 1.9 - 9.3% |
| 1,2-Propanediol | 0 - 9.4% |
| Enzymes | 0.0001 - 0.1% |
| Suds suppressor, dye, perfumes, water | Balance |

10) LIQUID AUTOMATIC DISHWASHING COMPOSITION

| Alcohol ethoxylate | 0 - 20% |
|---|---|
| Fatty acid ester sulphonate | 0 - 30% |
| Sodium dodecyl sulphate | 0 - 20% |
| Alkyl polyglycoside | 0 - 21% |
| Oleic acid | 0 - 10% |
| Sodium disilicate monohydrate | 18 - 33% |
| Sodium citrate dihydrate | 18 - 33% |
| Sodium stearate | 0 - 2.5% |
| Sodium perborate monohydrate | 0 - 13% |
| Tetraacetyl ethylene diamine (TAED) | 0 - 8% |
| Maleic acid/acrylic acid copolymer | 4 - 8% |

(continued)

| Enzymes | 0.0001 - 0.1% |
|---|---|

11) LIQUID AUTOMATIC DISHWASHING COMPOSITION CONTAINING PROTECTED BLEACH PARTICLES

| Sodium silicate | 5 - 10% |
|---|---|
| Tetrapotassium pyrophosphate | 15 - 25% |
| Sodium triphosphate | 0 - 2% |
| Potassium carbonate | 4 - 8% |
| Protected bleach particles, e.g. chlorine | 5 - 10% |
| Polymeric thickener | 0.7 - 1.5% |
| Potassium hydroxide | 0 - 2% |
| Enzymes | 0.0001 - 0.1% |
| Water | Balance |

11) Automatic dishwashing compositions as described in 1), 2), 3), 4), 6) and 10), wherein perborate is replaced by percarbonate.

12) Automatic dishwashing compositions as described in 1) - 6) which additionally contain a manganese catalyst. The manganese catalyst may, e.g., be one of the compounds described in "Efficient manganese catalysts for low-temperature bleaching", Nature 369, 1994, pp. 637-639.

Uses

The present invention is also directed to methods for using an alpha-amylase variant of the invention in detergents, in particular laundry detergent compositions and dishwashing detergent compositions, hard surface cleaning compositions, and in composition for desizing of textiles, fabrics or garments, for production of pulp and paper, beer making, ethanol production, and starch conversion processes as described above.

The present invention is further described by the following examples, which should not be construed as limiting the scope of the invention.

**MATERIALS & METHODS**

Enzymes:

KSM-K36: SEQ ID NO: 2, disclosed in EP 1,022,334 deposited as FERM BP 6945.
KSM-K38: SEQ ID NO: 4, disclosed in EP 1,022,334, deposited as FERM BP-6946.
*Bacillus subtilis* SHA273: Protease and amylase deleted *Bacillus subtilis* strain (disclosed in WO 95/10603).

Detergent:

Model detergent: A/P (Asia/Pacific) Model Detergent has the following composition: 20% STPP (sodium tripolyphosphate), 25% $Na_2SO_4$, 15% $Na_2CO_3$, 20% LAS (linear alkylbenzene sulfonate, Nansa 80S), 5% $C_{12}$-$C_{15}$ alcohol ethoxylate (Dobanol 25-7), 5% $Na_2Si_2O_5$, 0.3% NaCl.
Omo™ Multi Acao (Brazil),
Omo™ concentrated powder (EU) (Unilever)
Ariel Futur™ liquid (EU) (Procter and Gamble)
Commercial detergents containing alpha-amylase was inactivated by microwaves before wash.

Plasmids

pTVB110 is a plasmid replicating in *Bacillus subtilis* by the use of origin of replication from pUB110 (Gryczan, T.J. (1978), J. Bact. 134:318-329). The plasmid further encodes the *cat* gene, conferring resistance towards chlorampenicol, obtained from plasmid pC194 (Horinouchi, S. and Weisblum, B. (1982), J. Bact. 150: 815-825). The plasmid harbors a truncated version of the *Bacillus licheniformis* alpha-amylase gene, *amyL,* such that the *amyL* promoter, signal sequence and transcription terminator are present, but the plasmid does not provide an amy-plus phenotype (halo formation on starch containing agar). The alpha-amylase genes homologous to the KSM-K36 (SEQ ID NO: 1) and KSM-K38 (SEQ ID NO: 3) were cloned into the Pst1-Sal1 sites of pTVB110. The coding amylase gene was obtained by PCR reaction using purified genomic DNA from the Bacillus KSM-K36 strain as template and the DAX-8N (SEQ ID NO: 9) and DAX-8C (SEQ ID NO: 10) primers.

**Methods:**

General molecular biology methods:

Unless otherwise mentioned the DNA manipulations and transformations were performed using standard methods of molecular biology (Sambrook et al. (1989); Ausubel et al. (1995); Harwood and Cutting (1990).

Filter screening assays

The assay can be used to screening of alpha-amylase variants having an improved stability at high pH compared to the parent enzyme and alpha-amylase variants having an improved stability at high pH and medium temperatures compared to the parent enzyme depending of the screening temperature setting.

High pH filter assay

*Bacillus* libraries are plated on a sandwich of cellulose acetate (OE 67, Schleicher & Schuell, Dassel, Germany) - and nitrocellulose filters (Protran-Ba 85, Schleicher & Schuell, Dassel, Germany) on TY agar plates with 10 micro g/ml kanamycin at 37°C for at least 21 hours. The cellulose acetate layer is located on the TY agar plate.
Each filter sandwich is specifically marked with a needle after plating, but before incubation in order to be able to localize positive variants on the filter and the nitrocellulose filter with bound variants is transferred to a container with glycin-NaOH buffer, pH 8.6-10.6 and incubated at room temperature (can be altered from 10°-60°C) for 15 min. The cellulose acetate filters with colonies are stored on the TY-plates at room temperature until use. After incubation, residual activity is detected on plates containing 1% agarose, 0.2% starch in glycin-NaOH buffer, pH 8.6-10.6. The assay plates with nitrocellulose filters are marked the same way as the filter sandwich and incubated for 2 hours. at room temperature. After removal of the filters the assay plates are stained with 10% Lugol solution. Starch degrading variants are detected as white spots on dark blue background and then identified on the storage plates. Positive variants are rescreened twice under the same conditions as the first screen.

Low calcium filter assay

The *Bacillus* library are plated on a sandwich of cellulose acetate (OE 67, Schleicher & Schuell, Dassel, Germany) - and nitrocellulose filters (Protran-Ba 85, Schleicher & Schuell, Dassel, Germany) on TY agar plates with a relevant antibiotic, e.g., kanamycin or chloramphenicol, at 37°C for at least 21 hours. The cellulose acetate layer is located on the TY agar plate.
Each filter sandwich is specifically marked with a needle after plating, but before incubation in order to be able to localize positive variants on the filter and the nitrocellulose filter with bound variants is transferred to a container with carbonate/bicarbonate buffer pH 8.5-10 and with different EDTA concentrations (0.001 mM - 100 mM). The filters are incubated at room temperature for 1 hour. The cellulose acetate filters with colonies are stored on the TY-plates at room temperature until use. After incubation, residual activity is detected on plates containing 1% agarose, 0.2% starch in carbonate/bicarbonate buffer pH 8.5-10. The assay plates with nitrocellulose filters are marked the same way as the filter sandwich and incubated for 2 hours at room temperature. After removal of the filters the assay plates are stained with 10% Lugol solution. Starch degrading variants are detected as white spots on dark blue background and then identified on the storage plates. Positive variants are rescreened twice under the same conditions as the first screen.

Determination of Isoelectric Point

The pI is determined by isoelectric focusing (ex: Pharmacia, Ampholine, pH 3.5-9.3).

Fermentation of alpha-amylases and variants

Fermentation may be performed by methods well known in the art or as follows.

A *B. subtilis* strain harboring the relevant expression plasmid is streaked on a LB-agar plate with a relevant antibiotic, and grown overnight at 37°C.

The colonies are transferred to 100 ml BPX media supplemented with a relevant antibiotic

(for instance 10 mg/l chloroamphinicol) in a 500 ml shaking flask.

Composition of BPX medium:

| | | |
|---|---|---|
| Potato starch | 100 | g/l |
| Barley flour | 50 | g/l |
| BAN 5000 SKB | 0.1 | g/l |
| Sodium caseinate | 10 | g/l |
| Soy Bean Meal | 20 | g/l |
| $Na_2HPO_4, 12\,H_2O$ | 9 | g/l |
| Pluronic™ | 0.1 | g/l |

The culture is shaken at 37°C at 270 rpm for 4 to 5 days.

Cells and cell debris are removed from the fermentation broth by centrifugation at 4500 rpm in 20-25 minutes. Afterwards the supernatant is filtered to obtain a completely clear solution. The filtrate is concentrated and washed on an UF-filter (10000 cut off membrane) and the buffer is changed to 20mM Acetate pH 5.5. The UF-filtrate is applied on a S-sepharose F.F. and elution is carried out by step elution with 0.2 M NaCl in the same buffer. The eluate is dialysed against 10 mM Tris, pH 9.0 and applied on a Q-sepharose F.F. and eluted with a linear gradient from 0-0.3M NaCl over 6 column volumes. The fractions, which contain the activity (measured by the Phadebas assay) are pooled, pH was adjusted to pH 7.5 and remaining color was removed by a treatment with 0.5% W/vol. active coal in 5 minutes.

Stability determination

The amylase stability is measured using the method as follows: The enzyme is incubated under the relevant conditions. Samples are taken at various time points, e.g., after 0, 5, 10, 15 and 30 minutes and diluted 25 times (same dilution for all taken samples) in assay buffer (0.1M 50mM Britton buffer pH 7.3) and the activity is measured using the Phadebas assay (Pharmacia) under standard conditions pH 7.3, 37°C.

The activity measured before incubation (0 minutes) is used as reference (100%). The decline in percent is calculated as a function of the incubation time. The table shows the residual activity after, e.g., 30 minutes of incubation.

Measurement of the calcium- and pH-dependent stability

Normally industrial liquefaction processes runs using pH 6.0-6.2 as liquefaction pH and an addition of 40 ppm free calcium in order to improve the stability at 95°C-105°C. Some of the herein proposed substitutions have been made in order to improve the stability at

1. lower pH than pH 6.2 and/or
2. at free calcium levels lower than 40 ppm free calcium.

Two different methods can be used to measure the alterations in stability obtained by the different substitutions in the alpha-amylase in question:

Method 1. One assay which measures the stability at reduced pH, pH 5.0, in the presence of 5 ppm free calcium. 10 micro g of the variant are incubated under the following conditions: A 0.1 M acetate solution, pH adjusted to pH 5.0, containing 5 ppm calcium and 5% w/w common corn starch (free of calcium). Incubation is made in a water bath at 95°C for 30 minutes.

Method 2. One assay, which measure the stability in the absence of free calcium and where the pH is maintained at pH 6.0. This assay measures the decrease in calcium sensitivity:

10 micro g of the variant were incubated under the following conditions: A 0.1 M acetate solution, pH adjusted to pH 6.0, containing 5% w/w common corn starch (free of calcium). Incubation was made in a water bath at 95°C for 30 minutes.

Assays for Alpha-Amylase Activity

1. Phadebas assay

Alpha-amylase activity is determined by a method employing Phadebas® tablets as substrate. Phadebas tablets (Phadebas® Amylase Test, supplied by Pharmacia Diagnostic) contain a cross-linked insoluble blue-colored starch polymer, which has been mixed with bovine serum albumin and a buffer substance and tabletted.

For every single measurement one tablet is suspended in a tube containing 5 ml 50 mM Britton-Robinson buffer (50 mM acetic acid, 50 mM phosphoric acid, 50 mM boric acid, 0.1 mM $CaCl_2$, pH adjusted to the value of interest with NaOH). The test is performed in a water bath at the temperature of interest. The alpha-amylase to be tested is diluted in x ml of 50 mM Britton-Robinson buffer. 1 ml of this alpha-amylase solution is added to the 5 ml 50 mM Britton-Robinson buffer. The starch is hydrolyzed by the alpha-amylase giving soluble blue fragments. The absorbance of the resulting blue solution, measured spectrophotometrically at 620 nm, is a function of the alpha-amylase activity.

It is important that the measured 620 nm absorbance after 10 or 15 minutes of incubation (testing time) is in the range of 0.2 to 2.0 absorbance units at 620 nm. In this absorbance range there is linearity between activity and absorbance (Lambert-Beer law). The dilution of the enzyme must therefore be adjusted to fit this criterion. Under a specified set of conditions (temp., pH, reaction time, buffer conditions) 1 mg of a given alpha-amylase will hydrolyze a certain amount of substrate and a blue colour will be produced. The colour intensity is measured at 620 nm. The measured absorbance is directly proportional to the specific activity (activity/mg of pure alpha-amylase protein) of the alpha-amylase in question under the given set of conditions.

2. Alternative method

Alpha-amylase activity is determined by a method employing the PNP-G7 substrate. PNP-G7 which is a abbreviation for p-nitrophenyl-alpha,D-maltoheptaoside is a blocked oligosaccharide which can be cleaved by an endo-amylase. Following the cleavage, the alpha-Glucosidase included in the kit digest the substrate to liberate a free PNP molecule which has a yellow colour and thus can be measured by visible spectophometry at $\lambda=405nm$. (400-420 nm.). Kits containing PNP-G7 substrate and alpha-Glucosidase is manufactured by Boehringer-Mannheim (cat. No. 1054635).

To prepare the substrate one bottle of substrate (BM 1442309) is added to 5 ml buffer (BM1442309). To prepare the alpha-glucosidase one bottle of alpha-Glucosidase (BM 1462309) is added to 45 ml buffer (BM1442309). The working solution is made by mixing 5 ml alpha-Glucosidase solution with 0.5 ml substrate.

The assay is performed by transforming 20 micro I enzyme solution to a 96 well microtitre plate and incubating at 25°C. 200 micro I working solution, 25°C is added. The solution is mixed and pre-incubated 1 minute and absorption is measured every 15 secounds over 3 minutes at OD 405 nm.

The slope of the time dependent absorption-curve is directly proportional to the specific activity (activity per mg enzyme) of the alpha-amylase in question under the given set of conditions.

Specific activity determination

The specific activity is determined as activity/mg enzyme using one of the methods described above. The manufactures instructions are followed (see also below under "Assay for alpha-amylase activity).

Oxidation Stability determination

Raw filtered culture broths with different vatiants of the invention are diluted to an amylase activity of 100 KNU/ml (defined above) in 50 mM of a Britton-Robinson buffer at pH 9.0 and incubated at 40°C. Subsequently $H_2O_2$ is added to a concentration of 200 mM, and the pH value is re-adjusted to 9.0. The activity is now measured after 15 seconds and after 5, 15, and 30 minutes. The absorbance of the resulting blue solution, measured spectrophotometrically at 620 nm, is a function of the alpha-amylase activity.

Washing Performance

Washing performance is evaluated by washing soiled test swatches for 15 and 30 minutes at 25°C and 40°C, respectively; at a pH in the range from 9-10.5; water hardness in the range from 6 to 15 dH; Ca:Mg ratio of from 2:1 to 4:1, in different detergent solutions (see above as described above in the Materials section) dosed from 3 to 5 g/l dependent on the detergent with the alpha-amylase variant in question.

The recombinant alpha-amylase variant is added to the detergent solutions at concentrations of for instance 0.01-5 mg/l. The test swatches aree soiled with orange rice starch (CS-28 swatches available from CFT, Center for Test Material, Holland).

After washing, the swatches are evaluated by measuring the remission at 460 nm using an Elrepho Remission Spectrophotometer. The results are expressed as ΔR = remission of the swatch washed with the alpha-amylase minus the remission of a swatch washed at the same conditions without the alpha-amylase.

General method for random mutagenesis by use of the DOPE program

The random mutagenesis may be carried out as follows:

1. Select regions of interest for modification in the parent enzyme
2. Decide on mutation sites and non-mutated sites in the selected region
3. Decide on which kind of mutations should be carried out, e.g. with respect to the desired stability and/or performance of the variant to be constructed
4. Select structurally reasonable mutations.
5. Adjust the residues selected by step 3 with regard to step 4.
6. Analyze by use of a suitable dope algorithm the nucleotide distribution.
7. If necessary, adjust the wanted residues to genetic code realism (e.g., taking into account constraints resulting from the genetic code (e.g. in order to avoid introduction of stop codons))(the skilled person will be aware that some codon combinations cannot be used in practice and will need to be adapted)
8. Make primers
9. Perform random mutagenesis by use of the primers
10. Select resulting α-amylase variants by screening for the desired improved properties.

Suitable dope algorithms for use in step 6 are well known in the art. One algorithm is described by Tomandl, D. et al., Journal of Computer-Aided Molecular Design, 11 (1997), pp. 29-38). Another algorithm, DOPE, is described in the following:

The dope program

The "DOPE" program is a computer algorithm useful to optimize the nucleotide composition of a codon triplet in such a way that it encodes an amino acid distribution which resembles most the wanted amino acid distribution. In order to assess which of the possible distributions is the most similar to the wanted amino acid distribution, a scoring function is needed. In the "Dope" program the following function was found to be suited:

$$s \equiv \prod_{i=1}^{N} \left( \frac{x_i^{y_i} \left(1-x_i\right)^{1-y_i}}{y_i^{y_i} \left(1-y_i\right)^{1-y_i}} \right)^{w_i} ,$$

where the $x_i$'s are the obtained amounts of amino acids and groups of amino acids as calculated by the program, $y_i$'s are the wanted amounts of amino acids and groups of amino acids as defined by the user of the program (e.g. specify which of the 20 amino acids or stop codons are wanted to be introduced, e.g. with a certain percentage (e.g. 90% Ala, 3% Ile, 7% Val), and $w_i$'s are assigned weight factors as defined by the user of the program (e.g., depending on the importance of having a specific amino acid residue inserted into the position in question). N is 21 plus the number of amino acid groups as defined by the user of the program. For purposes of this function 0° is defined as being 1.

A Monte-Carlo algorithm (one example being the one described by Valleau, J.P. & Whittington, S.G. (1977) A guide to Mont Carlo for statistical mechanics: 1 Highways. In "Stastistical Mechanics, Part A" Equlibrium Techniqeues ed. B.J. Berne, New York: Plenum) is used for finding the maximum value of this function. In each iteration the following steps are performed:

1. A new random nucleotide composition is chosen for each base, where the absolute difference between the current and the new composition is smaller than or equal to d for each of the four nucleotides G,A,T,C in all three positions of the codon (see below for definition of d).
2. The scores of the new composition and the current composition are compared by the use of the function s as described above. If the new score is higher or equal to the score of the current composition, the new composition is kept and the current composition is changed to the new one. If the new score is smaller, the probability of keeping the new composition is exp(100(new_score-current_score)).

A cycle normally consists of 1000 iterations as described above in which d is decreasing linearly from 1 to 0. One hundred or more cycles are performed in an optimization process. The nucleotide composition resulting in the highest score is finally presented.

**EXAMPLES**

**Example 1**

**Construction of stabilised amylase variants**

Stabilising amino acid substitutions can be introduced by the mega-primer-PCR method described by Sarkar and Sommer, 1990, BioTechniques 8: 404-407, using a mutagenesis primer and two specific primers binding upstreams and down-streams, respectively of both the point of mutation and the restriction sites to be used for cloning.

To introduce the substitutions: E84Q, N96D, A315S, A445V, G464N, N121D and N393H the following mutagenesis primers could be used:

Primer Amrk752-E84Q:

(SEQ ID N0:11) ctaaggcacagctt**caa**cgagctattgggtcc

Primer Amrk752-N96D:

(SEQ ID NO:12) ccttaaatctaatgatatc**gat**gtatacggagatg

Primer Amrk752-A315S:

(SEQ ID NO:13) ttataatttttaccgg**tct**tcacaacaaggtgga

Primer Amrk752-A445V:

(SEQ ID NO:14) gtaggacgtcagaat**gta**ggacaaacatggac

Primer Amrk752-G464N:

(SEQ ID NO:15) ccgttacaattaat**aac**gatggatggggcgaattc

Primer Amrk230-N121D:

(SEQ ID NO:16) gcaagctgttcaagta**gat**ccaacgaatcgttgg

Primer Amrk230-N390H:

(SEQ ID NO:17) gcttgatgcacgtcaa**gat**tacgcatatggcacg

-where the mutated codon is highlighted.

The amylase variant Amrk752 can be constructed by simultaneous introduction of the first five substitutions into SEQ 4 while Amrk230 can be constructed by introducing the last to substitutions into SEQ 4.

In a similar manner can Amrk299 be constructed on the basis of SEQ 4 by introducing the substitutions: T125S, S144P, I173L, D210E, N393H, V408I, R442Q, N444H, Q448A and G464S.

Wild type and variant amylases could be expressed in B.subtilis strains deficient of background amylase and protease activity and following be purified by conventional purifications methods.

**Example 2:**

**Activity at alkaline pH**

The relative activity of the amylases at alkaline pH was measured on culture broth, and the activity at pH 8 was defined to be 100% for comparison of the results in the table below. The Phadebas amylase assay system manufacted by Pharmacia AB was used in pH 10 buffer at 50°C and with 15 min reaction time.

| Amylase | pH 8 | pH 10 |
|---------|------|-------|
| SEQ 4 | 100% | 6% |
| Amrk230 | 100% | 94% |
| Amrk299 | 100% | 19% |
| Amrk752 | 100% | 49% |

**Claims**

1.  A variant of a parent alpha-amylase, comprising an alteration at one or more positions selected from the group of:
    2,9,14,15,16,26,27,48,49,51,52,53,54,58,73,88,94,96,103,104,107,108,111,114,128,130,   133,138,140,142,144,
    148,149,156,161,165,166,168,171,173,174,178,179,180,181,183,184,187,    188,190,194,197,198,199,200,201,
    202,203,204,205,207,209,210,211,212,214,221,222,224,228,   230,233,234,237,239,241,242,252,253,254,   255,
    260,264,265,267,275,276,277,280,281,286,290,   293,301,305,314,315,318,329,333,340,341,356,375,376,   377,
    380,383,384,386,389,399,403,404, 405,406,427,441,444,453,454,472,479,480 wherein

    (a) the alteration(s) are independently

    (i) an insertion of an amino acid downstream of the amino acid which occupies the position,
    (ii) a deletion of the amino acid which occupies the position, or
    (iii) a substitution of the amino acid which occupies the position with a different amino acid,

    (b) the variant has alpha-amylase activity, and
    (c) each position corresponds to a position of the amino acid sequence of the parent alpha-amylase having the amino acid sequence of the KSM-36 alpaha-amylases shown in SEQ ID NO:2.

2.  The variant of claim 1, which variant has one or more of the following mutations:
    G2P,A; M9I,L,F; H14Y; L15M,I,F,T; E16P; H26Y,Q,R,N; D27N,S,T; G48A,V,S,T; N49X; Q51X; A52X; D53E,Q,R;
    V54X; A58V,L,I,F; V73L,I,F; E84Q; G88X; D94X; N96Q; M103I,L,F; N104D; M/L107G,A,V,T,S,I,L,F; G108A; F111G,
    A,V,I,L,T; A114D,I,L,M,V,R; T125S; D128T,E; S130T,C; Y133F,H; W138F,Y; G140H,R,K,D,N; D142H,R,K,N;
    S144P; N148S; A149I; R156H,K,D,N; N161X; W165R; D166E; R168P; E171L,I,F; H173R,K,L; I173L; L174I,F;
    A178N,Q,R,K,H; N179G,A,T,S; T180N,Q,R,K,H; N181X; N183X; W184R,K; D187N,S,T; E188P,T,I,S; N190F;
    D194X; L197X; G198X; S199X; N200X; I201L,M,F,Y; D202X; F203L,I,F,M; S204X; H205X; E207Y,R; Q209V,L,I,
    F,M; E210X; E211Q; L212I,F; D214N,R,K,H; D221 N; E222Q,T; D224N,Q; Y228F; L230I,F; I233A,V,L,F; K234N,
    Q; P237X; W239X; T241L,I,F,M; S242P,R; A252T; D253G,A,V,N; Q254K; D255N,Q,E,P; G260A; K264Q,S,T;
    D265N,Y; V267L,I,F,M; D275N,T; E276K; M277T,I,L,F; E280N,T,Q,S; M281H,I,L,F; V286X, preferably V286Y,L,I,
    F; Y290X; Y293H,F; S301G,A,D,K,E,R; R305A,K,Q,E,H,D,N; E314K,Q,R,S,T,H,N; A315K,R,S; I318L,M,F; T329S;
    E333Q; A340R,K,N,D,Q,E; D341P,T,S,Q,N; G356Q,E,S,T,A; S375P; A376S; K377L,I,F,M; M380I,L,F; E383P,Q;
    L384I,F; D386N,Q,R,K,I,L; Q389K,R; Y399A,D,H; W403X; D404N; I405L,F; V406I,L,F,A,D; N427X; H44IK,N,D,Q,
    E; R442Q; Q444E,K,R; A445V; Q448A; H453R,K,Q,N; A454S,T,P; G472R, N479Q,K,R; Q480K,R.

3.  The variant of claims 1 or 2, wherein the variant has the following mutations:
    N49I+L/M107A;  N49L+L/M107A;  G48A+N49I+L/M107A;  G48A+N49L+L/M107A;  E188S,T,P+N190F+I201F+
    K264S;
    G48A+N49I+L/M107A+E188S,T,P+N190F+I201F+K264S; N190F+I201F;N190F+K264S; I201F+K264S;G140H+
    D142H+R156H,Y(+S144P); G140K+D142D+R156H,Y(+S144P); L197M+G198Y+S199A; L15T+E188S+Q209V+
    A376S+G472R; G48A+N49I+L/M107A+G140H+D142H+R156H,Y+E188P+N190F+I201F+K264S(+S144P);
    N49T+L/M107A+G140H+D142H+R156H,Y+E188P+N190F+I201F+K264S(+S144P).

4.  The variant according to any of claims 1-3, wherein the parent alpha-amylase has an amino acid sequence which has a degree of identity to SEQ ID NO: 2 of at least 60%, preferably 70%, more preferably at least 80%, even more preferably at least about 90%, even more preferably at least 95%, even more preferably at least 97%, and even more preferably at least 99%.

5.  The variant of any of claims 1-4, wherein the parent alpha-amylase is encoded by a nucleic acid sequence, which hydridizes under medium, preferred high stringency conditions, with the nucleic acid sequence of SEQ ID NO: 1 or 3.

6.  The variant of claims 1-5, wherein the parent alpha-amylase is KSM-K38 shown in SEQ ID NO: 4.

7.  The variant of claims 1-6, which variant has altered pl, in particular a higher pl than the parent alpha-amylase.

8.  A DNA construct comprising a DNA sequence encoding an alpha-amylase variant according to any one of claims 1 to 7.

9.  A recombinant expression vector which carries a DNA construct according to claim 8.

**10.** A cell which is transformed with a DNA construct according to claim 8 or a vector according to claim 9.

**11.** A cell according to claim 10, which is a microorganism, preferably a bacterium or a fungus, in particular a gram-positive bacterium, such as *Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, Bacillus lautus* or *Bacillus thuringiensis.*

**12.** A detergent additive comprising an alpha-amylase variant according to any one of claims 1-7, optionally in the form of a non-dusting granulate, stabilised liquid or protected enzyme.

**13.** A detergent additive according to claim 12, which contains 0.02-200 mg of enzyme protein/g of the additive.

**14.** A detergent additive according to claims 12 or 13, which additionally comprises another enzyme such as a protease, a lipase, a peroxidase, a pectate lyase, an amylase, or another amylolytic enzyme, such as maltogenic alpha-amylase or glucoamylase, mannanase, CGTase, and/or a cellulase.

**15.** A detergent composition comprising an alpha-amylase variant according to any of claims 1-7.

**16.** A detergent composition according to claim 15, which additionally comprises another enzyme such as a protease, a lipase, a peroxidase, a pectate lyase another amylolytic enzyme, glucoamylase, CGTase, mannanase, maltogenic amylase, and/or a cellulase.

**17.** A manual or automatic dishwashing detergent composition comprising an alpha-amylase variant according to any of claims 1-7.

**18.** A dishwashing detergent composition according to claim 17, which additionally comprises another enzyme such as a protease, a lipase, a peroxidase, a pectate lyase, an amylase, or another amylolytic enzyme, such as glucoamylase, CGTase, mannanase, maltogenic amylase and/or a cellulase.

**19.** A manual or automatic laundry washing composition comprising an alpha-amylase variant according to any of claims 1-7.

**20.** A laundry washing composition according to claim 19, which additionally comprises another enzyme such as a protease, a lipase, a peroxidase, a pectate lyase, an amylase, and/or another amylolytic enzyme, such as glucoamylase, CGTase, mannanase, maltogenic amylase and/or a cellulase.

**21.** Use of an alpha-amylase variant according to any one of claims 1-7 or a composition or an additive according to claims 12 to 20 for washing and/or dishwashing.

**22.** Use of an alpha-amylase variant according to any one of claims 1-7 or a composition or an additive according to claims 12 to 20 for textile desizing.

**23.** Use of an alpha-amylase variant according to any of claims 1-7 or a composition or an additive according to claims 12 to 20 for starch liquefaction.

**24.** Use of an alpha-amylase variant according to any of claims 1-7 or a composition or an additive according to claims 12 to 20 for alcochol production, in particular ethanol production.

```
        1                                                        50
BLA     ANLNGTLMQY FEWYMPNDGQ HWKRLQNDSA YLAEHGITAV WIPPAYKGTS
KSM1378 HHNGTNGTMMQY FEWHLPNDGN HWNRLRDDAA NLKSKGITAV WIPPAWKGTS
K36     DGLNGTMMQY YEWHLENDGQ HWNRLHDDAE ALSNAGITAI WIPPAYKGNS
K38     DGLNGTMMQY YEWHLENDGQ HWNRLHDDAA ALSDAGITAI WIPPAYKGNS

        51                                                       100
BLA     QADVGYGAYD LYDLGEFHQK GTVRTKYGTK GELQSAIKSL HSRDINVYGD
KSM1378 QNDVGYGAYD LYDLGEFNQK GTVRTKYGTR SQLQGAVTSL KNNGIQVYGD
K36     QADVGYGAYD LYDLGEFNQK GTVRTKYGTK AQLERAIGSL KSNDINVYGD
K38     QADVGYGAYD LYDLGEFNQK GTVRTKYGTK AQLERAIGSL KSNDINVYGD

        101                                                      150
BLA     VVINHKGGAD ATEDVTAVEV DPADRNRVIS GEHLIKAWTH FHFPGRGSTY
KSM1378 VVMNHKGGAD GTEMVNAVEV NRSNRNQEIS GEYTIEAWTK FDFPGRGNTH
K36     VVMNHKLGAD FTEAVQAVQV NPSNRWQDIS GVYTIDAWTG FDFPGRNNAY
K38     VVMNHKMGAD FTEAVQAVQV NPTNRWQDIS GAYTIDAWTG FDFSGRNNAY

        151                                                      197
BLA     SDFKWHWYHF DGTDWDE.SR KLNRIYKFQG KA..WDWEVS NENGNYDYLM
KSM1378 SNFKWRWYHF DGTDWDQSRQ LQNKIYKFRG TGKAWDWEVD IENGNYDYLM
K36     SDFKWRWFHF NGVDWDQ.RY QENHLFRFAN TN..WNWRVD EENGNYDYLL
K38     SDFKWRWFHF NGVDWDQ.RY QENHIFRFAN TN..WNWRVD EENGNYDYLL

        198                                                      247
BLA     YADIDYDHPD VAAEIKRWGT WYANELQLDG FRLDAVKHIK FSFLRDWVNH
KSM1378 YADIDMDHPE VINELRNWGV WYTNTLNLDG FRIDAVKHIK YSYTRDWLTH
K36     GSNIDFSHPE VQEELKDWGS WFTDELDLDG YRLDAIKHIP FWYTSDWVRH
K38     GSNIDFSHPE VQDELKDWGS WFTDELDLDG YRLDAIKHIP FWYTSDWVRH

        248                                                      297
BLA     VREKTGKEMF TVAEYWQNDL GALENYLNKT NFNHSVFDVP LHYQFHAAST
KSM1378 VRNTTGKPMF AVAEFWKNDL AAIENYLNKT SWNHSVFDVP LHYNLYNASN
K36     QRSEADQDLF VVGEYWKDDV GALEFYLDEM NWEMSLFDVP LNYNFYRASK
K38     QRNEADQDLF VVGEYWKDDV GALEFYLDEM NWEMSLFDVP LNYNFYRASQ

        298                                                      347
BLA     QGGGYDMRKL LNGTVVSKHP LKSVTFVDNH DTQPGQSLES TVQTWFKPLA
KSM1378 SGGYRDMRNI LNGSVVQKHP IHAVTFVDNH DSQPGEALES FVQSWFKPLA
K36     QGGSYDMRNI LRGSLVEAHP IHAVTFVDNH DTQPGESLES WVADWFKPLA
K38     QGGSYDMRNI LRGSLVEAHP MHAVTFVDNH DTQPGESLES WVADWFKPLA

        348                                                      397
BLA     YAFILTRESG YPQVFYGDMY GTKGDSQREI PALKHKIEPI LKARKQYAYG
KSM1378 YALILTREQG YPSVFYGDYY GIPTHG...V PSMKSKIDPL LQARQTYAYG
K36     YATILTREGG YPNVFYGDYY GIPNDN...I SAKKDMIDEL LDARQNYAYG
K38     YATILTREGG YPNVFYGDYY GIPNDN...I SAKKDMIDEL LDARQNYAYG

        398                                                      447
BLA     AQHDYFDHHD IVGWTREGDS SVANSGLAAL ITDGPGGAKR MYVGRQNAGE
KSM1378 TQHDYFDHHD IIGWTREGDS SHPNSGLATI MSDGPGGNKW MYVGKHKAGQ
K36     TQHDYFDHWD IVGWTREGTS SRPNSGLATI MSNGPGGSKW MYVGQQHAGQ
K38     TQHDYFDHWD VVGWTREGSS SRPNSGLATI MSNGPGGSKW MYVGRQNAGQ

        448                                      483
BLA     TWHDITGNRS EPVVINSEGW GEFHVNGGSV SIYVQR
KSM1378 VWRDITGNRS GTVTINADGW GNFTVNGGAV SVWVKQ
K36     TWTDLTGNHA ASVTINGDGW GEFFTNGGSV SVYVNQ
K38     TWTDLTGNNG ASVTINGDGW GEFFTNGGSV SVYVNQ
```

## Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1022334 A
- WO 9700324 A
- EP 0252666 A
- US 4683202 A
- US 4760025 A
- WO 9522625 A
- WO 9600343 A
- WO 9117243 A
- EP 238023 A
- US 5231017 A
- US 3912590 A
- EP 252730 A
- EP 63909 A
- US 4335208 A
- US 4560651 A
- WO 9514807 A
- WO 9521247 A
- US 4643736 A
- EP 119920 A
- WO 8906279 A
- WO 8906270 A
- WO 9425583 A
- WO 9219729 A
- WO 9820115 A
- WO 9820116 A
- WO 9834946 A
- EP 258068 A
- EP 305216 A
- WO 9613580 A
- EP 218272 A
- EP 331376 A
- GB 1372034 A
- WO 9506720 A
- WO 9627002 A
- WO 9612012 A
- JP 64744992 B
- WO 9116422 A
- WO 9205249 A
- WO 9401541 A
- EP 407225 A
- EP 260105 A
- WO 9535381 A
- WO 9600292 A
- WO 9530744 A
- WO 9425578 A
- WO 9514783 A
- WO 9522615 A
- WO 9704079 A
- WO 9707202 A
- GB 1296839 A
- WO 9402597 A
- WO 9418314 A
- WO 9623873 A
- WO 9743424 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- DK 9800299 W
- WO 9324618 A
- WO 9510602 A
- WO 9815257 A
- WO 9927083 A
- WO 9927084 A
- US 4106991 A
- US 4661452 A
- GB 1483591 A
- EP 238216 A
- WO 9219709 A
- WO 9219708 A
- WO 9510603 A

### Non-patent literature cited in the description

- **TSUKAMOTO et al.** *Biochemical and Biophysical Research Communications,* 1988, vol. 151, 25-31
- **GABORIAUD et al.** *FEBS LETTERS,* 1987, vol. 224, 149-155

- **HUBER, T ; TORDA, AE.** *PROTEIN SCIENCE,* 1998, vol. 7 (1), 142-149
- **S.L. BEAUCAGE ; M.H. CARUTHERS.** *Tetrahedron Letters,* 1981, vol. 22, 1859-1869
- **MATTHES et al.** *The EMBO J.,* 1984, vol. 3, 801-805
- **R.K. SAIKI et al.** *Science,* 1988, vol. 239, 487-491
- **MORINAGA et al.** *Biotechnology,* 1984, vol. 2, 646-639
- **NELSON ; LONG.** *Analytical Biochemistry,* 1989, vol. 180, 147-151
- **LEUNG et al.** *Technique,* 1989, vol. 1, 11-15
- **FOWLER et al.** *Molec. Gen. Genet.,* 1974, vol. 133, 179-191
- **SAMBROOK et al.** Molecular Cloning: A Laboratory Manual. Cold Spring Harbor, 1989
- **DARTOIS et al.** *Biochemica et Biophysica Acta,* 1993, vol. 1131, 253-360
- Efficient manganese catalysts for low-temperature bleaching. *Nature,* 1994, vol. 369, 637-639
- **GRYCZAN, T.J.** *J. Bact.,* 1978, vol. 134, 318-329
- **HORINOUCHI, S. ; WEISBLUM, B.** *J. Bact.,* 1982, vol. 150, 815-825
- **TOMANDL, D. et al.** *Journal of Computer-Aided Molecular Design,* 1997, vol. 11, 29-38
- A guide to Mont Carlo for statistical mechanics: 1 Highways. **VALLEAU, J.P. ; WHITTINGTON, S.G.** Stastistical Mechanics, Part A'' Equlibrium Techniqeues. Plenum, 1977
- **SARKAR ; SOMMER.** *BioTechniques,* 1990, vol. 8, 404-407

SEQUENCE LISTING

<110> Andersen, Carsten

<120> Alpha-amylase variant with altered properties

<130> 10115.215-EP

<160> 17

<170> PatentIn version 3.2

<210> 1
<211> 1650
<212> DNA
<213> Bacillus sp.


<220>
<221> CDS
<222> (65)..(1567)

<220>
<221> sig_peptide
<222> (65)..(128)

<220>
<221> mat_peptide
<222> (128)..()

<400> 1
cttgaatcat tatttaaagc tggttatgat atatgtaagc gttatcatta aaaggaggta        60

```
tttg atg aaa aga tgg gta gta gca atg ctg gca gtg tta ttt tta ttt       109
     Met Lys Arg Trp Val Val Ala Met Leu Ala Val Leu Phe Leu Phe
     -20              -15              -10

cct tcg gta gta gtt gca gat ggc ttg aat gga acg atg atg cag tat         157
Pro Ser Val Val Val Ala Asp Gly Leu Asn Gly Thr Met Met Gln Tyr
    -5               -1  1               5                   10

tat gag tgg cat cta gag aat gat ggg caa cac tgg aat cgg ttg cat         205
Tyr Glu Trp His Leu Glu Asn Asp Gly Gln His Trp Asn Arg Leu His
                15               20                  25

gat gat gcc gaa gct tta agt aat gcg ggt att aca gct att tgg ata         253
Asp Asp Ala Glu Ala Leu Ser Asn Ala Gly Ile Thr Ala Ile Trp Ile
            30               35                  40

ccc cca gcc tac aaa gga aat agt cag gct gat gtt ggg tat ggt gca         301
Pro Pro Ala Tyr Lys Gly Asn Ser Gln Ala Asp Val Gly Tyr Gly Ala
        45               50               55

tac gac ctt tat gat tta ggg gag ttt aat caa aaa ggt acc gtt cga         349
Tyr Asp Leu Tyr Asp Leu Gly Glu Phe Asn Gln Lys Gly Thr Val Arg
    60               65               70

acg aaa tac ggg aca aag gct cag ctt gag cga gct ata ggg tcc cta         397
Thr Lys Tyr Gly Thr Lys Ala Gln Leu Glu Arg Ala Ile Gly Ser Leu
75               80               85                  90

aag tcg aat gat atc aat gtt tat ggg gat gtc gta atg aat cat aaa         445
Lys Ser Asn Asp Ile Asn Val Tyr Gly Asp Val Val Met Asn His Lys
                95               100                 105

tta gga gct gat ttc acg gag gca gtg caa gct gtt caa gta aat cct         493
Leu Gly Ala Asp Phe Thr Glu Ala Val Gln Ala Val Gln Val Asn Pro
            110              115                 120
```

```
tcg aac cgt tgg cag gat att tca ggt gtc tac acg att gat gca tgg    541
Ser Asn Arg Trp Gln Asp Ile Ser Gly Val Tyr Thr Ile Asp Ala Trp
        125             130             135

acg gga ttt gac ttt cca ggg cgc aac aat gcc tat tcc gat ttt aaa    589
Thr Gly Phe Asp Phe Pro Gly Arg Asn Asn Ala Tyr Ser Asp Phe Lys
        140             145             150

tgg aga tgg ttc cat ttt aat ggc gtt gac tgg gat caa cgc tat caa    637
Trp Arg Trp Phe His Phe Asn Gly Val Asp Trp Asp Gln Arg Tyr Gln
155             160             165             170

gaa aac cat ctt ttt cgc ttt gca aat acg aac tgg aac tgg cga gtg    685
Glu Asn His Leu Phe Arg Phe Ala Asn Thr Asn Trp Asn Trp Arg Val
            175             180             185

gat gaa gag aat ggt aat tat gac tat tta tta gga tcg aac att gac    733
Asp Glu Glu Asn Gly Asn Tyr Asp Tyr Leu Leu Gly Ser Asn Ile Asp
            190             195             200

ttt agc cac cca gag gtt caa gag gaa tta aag gat tgg ggg agc tgg    781
Phe Ser His Pro Glu Val Gln Glu Glu Leu Lys Asp Trp Gly Ser Trp
        205             210             215

ttt acg gat gag cta gat tta gat ggg tat cga ttg gat gct att aag    829
Phe Thr Asp Glu Leu Asp Leu Asp Gly Tyr Arg Leu Asp Ala Ile Lys
        220             225             230

cat att cca ttc tgg tat acg tca gat tgg gtt agg cat cag cga agt    877
His Ile Pro Phe Trp Tyr Thr Ser Asp Trp Val Arg His Gln Arg Ser
235             240             245             250

gaa gca gac caa gat tta ttt gtc gta ggg gag tat tgg aag gat gac    925
Glu Ala Asp Gln Asp Leu Phe Val Val Gly Glu Tyr Trp Lys Asp Asp
            255             260             265

gta ggt gct ctc gaa ttt tat tta gat gaa atg aat tgg gag atg tct    973
Val Gly Ala Leu Glu Phe Tyr Leu Asp Glu Met Asn Trp Glu Met Ser
        270             275             280

cta ttc gat gtt ccg ctc aat tat aat ttt tac cgg gct tca aag caa    1021
Leu Phe Asp Val Pro Leu Asn Tyr Asn Phe Tyr Arg Ala Ser Lys Gln
        285             290             295

ggc gga agc tat gat atg cgt aat att tta cga gga tct tta gta gaa    1069
Gly Gly Ser Tyr Asp Met Arg Asn Ile Leu Arg Gly Ser Leu Val Glu
    300             305             310

gca cat ccg att cat gca gtt acg ttt gtt gat aat cat gat act cag    1117
Ala His Pro Ile His Ala Val Thr Phe Val Asp Asn His Asp Thr Gln
315             320             325             330

cca gga gag tca tta gaa tca tgg gtc gct gat tgg ttt aag cca ctt    1165
Pro Gly Glu Ser Leu Glu Ser Trp Val Ala Asp Trp Phe Lys Pro Leu
            335             340             345

gct tat gcg aca atc ttg acg cgt gaa ggt ggt tat cca aat gta ttt    1213
Ala Tyr Ala Thr Ile Leu Thr Arg Glu Gly Gly Tyr Pro Asn Val Phe
            350             355             360

tac ggt gac tac tat ggg att cct aac gat aac att tca gct aag aag    1261
Tyr Gly Asp Tyr Tyr Gly Ile Pro Asn Asp Asn Ile Ser Ala Lys Lys
        365             370             375

gat atg att gat gag ttg ctt gat gca cgt caa aat tac gca tat ggc    1309
Asp Met Ile Asp Glu Leu Leu Asp Ala Arg Gln Asn Tyr Ala Tyr Gly
        380             385             390

aca caa cat gac tat ttt gat cat tgg gat atc gtt gga tgg aca aga    1357
```

28

```
Thr Gln His Asp Tyr Phe Asp His Trp Asp Ile Val Gly Trp Thr Arg
395             400             405             410

gaa ggt aca tcc tca cgt cct aat tcg ggt ctt gct act att atg tcc     1405
Glu Gly Thr Ser Ser Arg Pro Asn Ser Gly Leu Ala Thr Ile Met Ser
                415             420             425

aat ggt cct gga gga tca aaa tgg atg tac gta gga cag caa cat gca     1453
Asn Gly Pro Gly Gly Ser Lys Trp Met Tyr Val Gly Gln Gln His Ala
                430             435             440

gga caa acg tgg aca gat tta act ggc aat cac gcg gcg tcg gtt acg     1501
Gly Gln Thr Trp Thr Asp Leu Thr Gly Asn His Ala Ala Ser Val Thr
                445             450             455

att aat ggt gat ggc tgg ggc gaa ttc ttt aca aat gga gga tct gta     1549
Ile Asn Gly Asp Gly Trp Gly Glu Phe Phe Thr Asn Gly Gly Ser Val
                460             465             470

tcc gtg tat gtg aac caa taataaaaag ccttgagaag ggattcctcc           1597
Ser Val Tyr Val Asn Gln
475             480

ctaactcaag gctttcttta tgtcgtttag ctcaacgctt ctacgaagct tta          1650
```

<210> 2
<211> 501
<212> PRT
<213> Bacillus sp.

<400> 2

```
Met Lys Arg Trp Val Val Ala Met Leu Ala Val Leu Phe Leu Phe Pro
    -20             -15             -10

Ser Val Val Val Ala Asp Gly Leu Asn Gly Thr Met Met Gln Tyr Tyr
-5              -1  1           5               10

Glu Trp His Leu Glu Asn Asp Gly Gln His Trp Asn Arg Leu His Asp
            15              20              25

Asp Ala Glu Ala Leu Ser Asn Ala Gly Ile Thr Ala Ile Trp Ile Pro
            30              35              40

Pro Ala Tyr Lys Gly Asn Ser Gln Ala Asp Val Gly Tyr Gly Ala Tyr
            45              50              55

Asp Leu Tyr Asp Leu Gly Glu Phe Asn Gln Lys Gly Thr Val Arg Thr
60              65              70              75

Lys Tyr Gly Thr Lys Ala Gln Leu Glu Arg Ala Ile Gly Ser Leu Lys
            80              85              90

Ser Asn Asp Ile Asn Val Tyr Gly Asp Val Val Met Asn His Lys Leu
            95              100             105

Gly Ala Asp Phe Thr Glu Ala Val Gln Ala Val Gln Val Asn Pro Ser
            110             115             120
```

Asn Arg Trp Gln Asp Ile Ser Gly Val Tyr Thr Ile Asp Ala Trp Thr
125 130 135

Gly Phe Asp Phe Pro Gly Arg Asn Asn Ala Tyr Ser Asp Phe Lys Trp
140 145 150 155

Arg Trp Phe His Phe Asn Gly Val Asp Trp Asp Gln Arg Tyr Gln Glu
160 165 170

Asn His Leu Phe Arg Phe Ala Asn Thr Asn Trp Asn Trp Arg Val Asp
175 180 185

Glu Glu Asn Gly Asn Tyr Asp Tyr Leu Leu Gly Ser Asn Ile Asp Phe
190 195 200

Ser His Pro Glu Val Gln Glu Glu Leu Lys Asp Trp Gly Ser Trp Phe
205 210 215

Thr Asp Glu Leu Asp Leu Asp Gly Tyr Arg Leu Asp Ala Ile Lys His
220 225 230 235

Ile Pro Phe Trp Tyr Thr Ser Asp Trp Val Arg His Gln Arg Ser Glu
240 245 250

Ala Asp Gln Asp Leu Phe Val Val Gly Glu Tyr Trp Lys Asp Asp Val
255 260 265

Gly Ala Leu Glu Phe Tyr Leu Asp Glu Met Asn Trp Glu Met Ser Leu
270 275 280

Phe Asp Val Pro Leu Asn Tyr Asn Phe Tyr Arg Ala Ser Lys Gln Gly
285 290 295

Gly Ser Tyr Asp Met Arg Asn Ile Leu Arg Gly Ser Leu Val Glu Ala
300 305 310 315

His Pro Ile His Ala Val Thr Phe Val Asp Asn His Asp Thr Gln Pro
320 325 330

Gly Glu Ser Leu Glu Ser Trp Val Ala Asp Trp Phe Lys Pro Leu Ala
335 340 345

Tyr Ala Thr Ile Leu Thr Arg Glu Gly Gly Tyr Pro Asn Val Phe Tyr
350 355 360

Gly Asp Tyr Tyr Gly Ile Pro Asn Asp Asn Ile Ser Ala Lys Lys Asp
365 370 375

Met Ile Asp Glu Leu Leu Asp Ala Arg Gln Asn Tyr Ala Tyr Gly Thr
380 385 390 395

Gln His Asp Tyr Phe Asp His Trp Asp Ile Val Gly Trp Thr Arg Glu

```
                    400                      405                      410

Gly Thr Ser Ser Arg Pro Asn Ser Gly Leu Ala Thr Ile Met Ser Asn
            415                 420                 425

Gly Pro Gly Gly Ser Lys Trp Met Tyr Val Gly Gln Gln His Ala Gly
        430                 435                 440

Gln Thr Trp Thr Asp Leu Thr Gly Asn His Ala Ala Ser Val Thr Ile
        445                 450                 455

Asn Gly Asp Gly Trp Gly Glu Phe Phe Thr Asn Gly Gly Ser Val Ser
460                 465                 470                 475

Val Tyr Val Asn Gln
                480


<210>   3
<211>   1745
<212>   DNA
<213>   Bacillus


<220>
<221>   CDS
<222>   (190)..(1692)

<220>
<221>   sig_peptide
<222>   (190)..(253)

<220>
<221>   mat_peptide
<222>   (253)..()

<400>   3
aactaagtaa catcgattca ggataaaagt atgcgaaacg atgcgcaaaa ctgcgcaact      60

actagcactc ttcagggact aaaccacctt ttttccaaaa atgacatcat ataaacaaat     120

ttgtctacca atcactattt aaagctgttt atgatatatg taagcgttat cattaaaagg     180

aggtatttg atg aga aga tgg gta gta gca atg ttg gca gtg tta ttt tta     231
            Met Arg Arg Trp Val Val Ala Met Leu Ala Val Leu Phe Leu
            -20                 -15                 -10

ttt cct tcg gta gta gtt gca gat gga ttg aac ggt acg atg atg cag     279
Phe Pro Ser Val Val Val Ala Asp Gly Leu Asn Gly Thr Met Met Gln
        -5                -1  1                  5

tat tat gag tgg cat ttg gaa aac gac ggg cag cat tgg aat cgg ttg     327
Tyr Tyr Glu Trp His Leu Glu Asn Asp Gly Gln His Trp Asn Arg Leu
10                  15                  20                  25

cac gat gat gcc gca gct ttg agt gat gct ggt att aca gct att tgg     375
His Asp Asp Ala Ala Ala Leu Ser Asp Ala Gly Ile Thr Ala Ile Trp
                    30                  35                  40

att ccg cca gcc tac aaa ggt aat agt cag gcg gat gtt ggg tac ggt     423
Ile Pro Pro Ala Tyr Lys Gly Asn Ser Gln Ala Asp Val Gly Tyr Gly
                45                  50                  55

gca tac gat ctt tat gat tta gga gag ttc aat caa aag ggt act gtt     471
```

```
Ala Tyr Asp Leu Tyr Asp Leu Gly Glu Phe Asn Gln Lys Gly Thr Val
        60                  65                  70

cga acg aaa tac gga act aag gca cag ctt gaa cga gct att ggg tcc     519
Arg Thr Lys Tyr Gly Thr Lys Ala Gln Leu Glu Arg Ala Ile Gly Ser
    75                  80                  85

ctt aaa tct aat gat atc aat gta tac gga gat gtc gtg atg aat cat     567
Leu Lys Ser Asn Asp Ile Asn Val Tyr Gly Asp Val Val Met Asn His
90                  95                  100                 105

aaa atg gga gct gat ttt acg gag gca gtg caa gct gtt caa gta aat     615
Lys Met Gly Ala Asp Phe Thr Glu Ala Val Gln Ala Val Gln Val Asn
                110                 115                 120

cca acg aat cgt tgg cag gat att tca ggt gcc tac acg att gat gcg     663
Pro Thr Asn Arg Trp Gln Asp Ile Ser Gly Ala Tyr Thr Ile Asp Ala
                125                 130                 135

tgg acg ggt ttc gac ttt tca ggg cgt aac aac gcc tat tca gat ttt     711
Trp Thr Gly Phe Asp Phe Ser Gly Arg Asn Asn Ala Tyr Ser Asp Phe
            140                 145                 150

aag tgg aga tgg ttc cat ttt aat ggt gtt gac tgg gat cag cgc tat     759
Lys Trp Arg Trp Phe His Phe Asn Gly Val Asp Trp Asp Gln Arg Tyr
        155                 160                 165

caa gaa aat cat att ttc cgc ttt gca aat acg aac tgg aac tgg cga     807
Gln Glu Asn His Ile Phe Arg Phe Ala Asn Thr Asn Trp Asn Trp Arg
170                 175                 180                 185

gtg gat gaa gag aac ggt aat tat gat tac ctg tta gga tcg aat atc     855
Val Asp Glu Glu Asn Gly Asn Tyr Asp Tyr Leu Leu Gly Ser Asn Ile
                190                 195                 200

gac ttt agt cat cca gaa gta caa gat gag ttg aag gat tgg ggt agc     903
Asp Phe Ser His Pro Glu Val Gln Asp Glu Leu Lys Asp Trp Gly Ser
            205                 210                 215

tgg ttt acc gat gag tta gat ttg gat ggt tat cgt tta gat gct att     951
Trp Phe Thr Asp Glu Leu Asp Leu Asp Gly Tyr Arg Leu Asp Ala Ile
            220                 225                 230

aaa cat att cca ttc tgg tat aca tct gat tgg gtt cgg cat cag cgc     999
Lys His Ile Pro Phe Trp Tyr Thr Ser Asp Trp Val Arg His Gln Arg
        235                 240                 245

aac gaa gca gat caa gat tta ttt gtc gta ggg gaa tat tgg aag gat     1047
Asn Glu Ala Asp Gln Asp Leu Phe Val Val Gly Glu Tyr Trp Lys Asp
250                 255                 260                 265

gac gta ggt gct ctc gaa ttt tat tta gat gaa atg aat tgg gag atg     1095
Asp Val Gly Ala Leu Glu Phe Tyr Leu Asp Glu Met Asn Trp Glu Met
                270                 275                 280

tct cta ttc gat gtt cca ctt aat tat aat ttt tac cgg gct tca caa     1143
Ser Leu Phe Asp Val Pro Leu Asn Tyr Asn Phe Tyr Arg Ala Ser Gln
            285                 290                 295

caa ggt gga agc tat gat atg cgt aat att tta cga gga tct tta gta     1191
Gln Gly Gly Ser Tyr Asp Met Arg Asn Ile Leu Arg Gly Ser Leu Val
            300                 305                 310

gaa gcg cat ccg atg cat gca gtt acg ttt gtt gat aat cat gat act     1239
Glu Ala His Pro Met His Ala Val Thr Phe Val Asp Asn His Asp Thr
        315                 320                 325

cag cca ggg gag tca tta gag tca tgg gtt gct gat tgg ttt aag cca     1287
Gln Pro Gly Glu Ser Leu Glu Ser Trp Val Ala Asp Trp Phe Lys Pro
```

```
                330                         335                              340                           345
          ctt gct tat gcg aca att ttg acg cgt gaa ggt ggt tat cca aat gta   1335
          Leu Ala Tyr Ala Thr Ile Leu Thr Arg Glu Gly Gly Tyr Pro Asn Val
                            350                 355                 360

          ttt tac ggt gat tac tat ggg att cct aac gat aac att tca gct aaa   1383
          Phe Tyr Gly Asp Tyr Tyr Gly Ile Pro Asn Asp Asn Ile Ser Ala Lys
                        365                 370                 375

          aaa gat atg att gat gag ctg ctt gat gca cgt caa aat tac gca tat   1431
          Lys Asp Met Ile Asp Glu Leu Leu Asp Ala Arg Gln Asn Tyr Ala Tyr
                        380                 385                 390

          ggc acg cag cat gac tat ttt gat cat tgg gat gtt gta gga tgg act   1479
          Gly Thr Gln His Asp Tyr Phe Asp His Trp Asp Val Val Gly Trp Thr
                        395                 400                 405

          agg gaa gga tct tcc tcc aga cct aat tca ggc ctt gcg act att atg   1527
          Arg Glu Gly Ser Ser Ser Arg Pro Asn Ser Gly Leu Ala Thr Ile Met
          410                 415                 420                 425

          tcg aat gga cct ggt ggt tcc aag tgg atg tat gta gga cgt cag aat   1575
          Ser Asn Gly Pro Gly Gly Ser Lys Trp Met Tyr Val Gly Arg Gln Asn
                            430                 435                 440

          gca gga caa aca tgg aca gat tta act ggt aat aac gga gcg tcc gtt   1623
          Ala Gly Gln Thr Trp Thr Asp Leu Thr Gly Asn Asn Gly Ala Ser Val
                            445                 450                 455

          aca att aat ggc gat gga tgg ggc gaa ttc ttt acg aat gga gga tct   1671
          Thr Ile Asn Gly Asp Gly Trp Gly Glu Phe Phe Thr Asn Gly Gly Ser
                        460                 465                 470

          gta tcc gtg tac gtg aac caa taacaaaaag ccttgagaag ggattcctcc     1722
          Val Ser Val Tyr Val Asn Gln
                        475                 480

          ctaactcaag gctttcttta tgt                                        1745


          <210>   4
          <211>   501
          <212>   PRT
          <213>   Bacillus

          <400>   4

          Met Arg Arg Trp Val Val Ala Met Leu Ala Val Leu Phe Leu Phe Pro
                -20                 -15                 -10

          Ser Val Val Val Ala Asp Gly Leu Asn Gly Thr Met Met Gln Tyr Tyr
          -5                  -1  1               5                   10

          Glu Trp His Leu Glu Asn Asp Gly Gln His Trp Asn Arg Leu His Asp
                        15                  20                  25

          Asp Ala Ala Ala Leu Ser Asp Ala Gly Ile Thr Ala Ile Trp Ile Pro
                        30                  35                  40

          Pro Ala Tyr Lys Gly Asn Ser Gln Ala Asp Val Gly Tyr Gly Ala Tyr
                        45                  50                  55

          Asp Leu Tyr Asp Leu Gly Glu Phe Asn Gln Lys Gly Thr Val Arg Thr
```

```
            60                      65                      70                      75

    Lys Tyr Gly Thr Lys Ala Gln Leu Glu Arg Ala Ile Gly Ser Leu Lys
                    80                      85                      90

    Ser Asn Asp Ile Asn Val Tyr Gly Asp Val Val Met Asn His Lys Met
                    95                     100                     105

    Gly Ala Asp Phe Thr Glu Ala Val Gln Ala Val Gln Val Asn Pro Thr
                   110                     115                     120

    Asn Arg Trp Gln Asp Ile Ser Gly Ala Tyr Thr Ile Asp Ala Trp Thr
           125                     130                     135

    Gly Phe Asp Phe Ser Gly Arg Asn Asn Ala Tyr Ser Asp Phe Lys Trp
    140                     145                     150                     155

    Arg Trp Phe His Phe Asn Gly Val Asp Trp Asp Gln Arg Tyr Gln Glu
                   160                     165                     170

    Asn His Ile Phe Arg Phe Ala Asn Thr Asn Trp Asn Trp Arg Val Asp
                   175                     180                     185

    Glu Glu Asn Gly Asn Tyr Asp Tyr Leu Leu Gly Ser Asn Ile Asp Phe
           190                     195                     200

    Ser His Pro Glu Val Gln Asp Glu Leu Lys Asp Trp Gly Ser Trp Phe
           205                     210                     215

    Thr Asp Glu Leu Asp Leu Asp Gly Tyr Arg Leu Asp Ala Ile Lys His
    220                     225                     230                     235

    Ile Pro Phe Trp Tyr Thr Ser Asp Trp Val Arg His Gln Arg Asn Glu
                   240                     245                     250

    Ala Asp Gln Asp Leu Phe Val Val Gly Glu Tyr Trp Lys Asp Asp Val
                   255                     260                     265

    Gly Ala Leu Glu Phe Tyr Leu Asp Glu Met Asn Trp Glu Met Ser Leu
           270                     275                     280

    Phe Asp Val Pro Leu Asn Tyr Asn Phe Tyr Arg Ala Ser Gln Gln Gly
           285                     290                     295

    Gly Ser Tyr Asp Met Arg Asn Ile Leu Arg Gly Ser Leu Val Glu Ala
    300                     305                     310                     315

    His Pro Met His Ala Val Thr Phe Val Asp Asn His Asp Thr Gln Pro
                   320                     325                     330

    Gly Glu Ser Leu Glu Ser Trp Val Ala Asp Trp Phe Lys Pro Leu Ala
           335                     340                     345
```

```
Tyr Ala Thr Ile Leu Thr Arg Glu Gly Gly Tyr Pro Asn Val Phe Tyr
        350             355             360
```

```
Gly Asp Tyr Tyr Gly Ile Pro Asn Asp Asn Ile Ser Ala Lys Lys Asp
        365             370             375
```

```
Met Ile Asp Glu Leu Leu Asp Ala Arg Gln Asn Tyr Ala Tyr Gly Thr
380             385             390             395
```

```
Gln His Asp Tyr Phe Asp His Trp Asp Val Val Gly Trp Thr Arg Glu
            400             405             410
```

```
Gly Ser Ser Ser Arg Pro Asn Ser Gly Leu Ala Thr Ile Met Ser Asn
        415             420             425
```

```
Gly Pro Gly Gly Ser Lys Trp Met Tyr Val Gly Arg Gln Asn Ala Gly
        430             435             440
```

```
Gln Thr Trp Thr Asp Leu Thr Gly Asn Asn Gly Ala Ser Val Thr Ile
        445             450             455
```

```
Asn Gly Asp Gly Trp Gly Glu Phe Phe Thr Asn Gly Gly Ser Val Ser
460             465             470             475
```

```
Val Tyr Val Asn Gln
            480
```

```
<210>   5
<211>   1920
<212>   DNA
<213>   Bacillus licheniformis
```

```
<220>
<221>   CDS
<222>   (421)..(1872)
```

```
<400>   5
cggaagattg gaagtacaaa aataagcaaa agattgtcaa tcatgtcatg agccatgcgg      60

gagacggaaa aatcgtctta atgcacgata tttatgcaac gttcgcagat gctgctgaag     120

agattattaa aaagctgaaa gcaaaaggct atcaattggt aactgtatct cagcttgaag     180

aagtgaagaa gcagagaggc tattgaataa atgagtagaa gcgccatatc ggcgcttttc     240

ttttggaaga aaatataggg aaaatggtac ttgttaaaaa ttcggaatat ttatacaaca     300

tcatatgttt cacattgaaa ggggaggaga atcatgaaac aacaaaaacg gctttacgcc     360

cgattgctga cgctgttatt tgcgctcatc ttcttgctgc ctcattctgc agcagcggcg     420
```

```
gca aat ctt aat ggg acg ctg atg cag tat ttt gaa tgg tac atg ccc     468
Ala Asn Leu Asn Gly Thr Leu Met Gln Tyr Phe Glu Trp Tyr Met Pro
1               5                   10                  15
```

```
aat gac ggc caa cat tgg agg cgt ttg caa aac gac tcg gca tat ttg     516
Asn Asp Gly Gln His Trp Arg Arg Leu Gln Asn Asp Ser Ala Tyr Leu
```

```
                20                        25                        30
      gct gaa cac ggt att act gcc gtc tgg att ccc ccg gca tat aag gga    564
      Ala Glu His Gly Ile Thr Ala Val Trp Ile Pro Pro Ala Tyr Lys Gly
              35              40              45

      acg agc caa gcg gat gtg ggc tac ggt gct tac gac ctt tat gat tta    612
      Thr Ser Gln Ala Asp Val Gly Tyr Gly Ala Tyr Asp Leu Tyr Asp Leu
          50              55              60

      ggg gag ttt cat caa aaa ggg acg gtt cgg aca aag tac ggc aca aaa    660
      Gly Glu Phe His Gln Lys Gly Thr Val Arg Thr Lys Tyr Gly Thr Lys
      65              70              75              80

      gga gag ctg caa tct gcg atc aaa agt ctt cat tcc cgc gac att aac    708
      Gly Glu Leu Gln Ser Ala Ile Lys Ser Leu His Ser Arg Asp Ile Asn
                  85              90              95

      gtt tac ggg gat gtg gtc atc aac cac aaa ggc ggc gct gat gcg acc    756
      Val Tyr Gly Asp Val Val Ile Asn His Lys Gly Gly Ala Asp Ala Thr
                  100             105             110

      gaa gat gta acc gcg gtt gaa gtc gat ccc gct gac cgc aac cgc gta    804
      Glu Asp Val Thr Ala Val Glu Val Asp Pro Ala Asp Arg Asn Arg Val
              115             120             125

      att tca gga gaa cac cta att aaa gcc tgg aca cat ttt cat ttt ccg    852
      Ile Ser Gly Glu His Leu Ile Lys Ala Trp Thr His Phe His Phe Pro
          130             135             140

      ggg cgc ggc agc aca tac agc gat ttt aaa tgg cat tgg tac cat ttt    900
      Gly Arg Gly Ser Thr Tyr Ser Asp Phe Lys Trp His Trp Tyr His Phe
      145             150             155             160

      gac gga acc gat tgg gac gag tcc cga aag ctg aac cgc atc tat aag    948
      Asp Gly Thr Asp Trp Asp Glu Ser Arg Lys Leu Asn Arg Ile Tyr Lys
                  165             170             175

      ttt caa gga aag gct tgg gat tgg gaa gtt tcc aat gaa aac ggc aac    996
      Phe Gln Gly Lys Ala Trp Asp Trp Glu Val Ser Asn Glu Asn Gly Asn
                  180             185             190

      tat gat tat ttg atg tat gcc gac atc gat tat gac cat cct gat gtc    1044
      Tyr Asp Tyr Leu Met Tyr Ala Asp Ile Asp Tyr Asp His Pro Asp Val
                  195             200             205

      gca gca gaa att aag aga tgg ggc act tgg tat gcc aat gaa ctg caa    1092
      Ala Ala Glu Ile Lys Arg Trp Gly Thr Trp Tyr Ala Asn Glu Leu Gln
          210             215             220

      ttg gac ggt ttc cgt ctt gat gct gtc aaa cac att aaa ttt tct ttt    1140
      Leu Asp Gly Phe Arg Leu Asp Ala Val Lys His Ile Lys Phe Ser Phe
      225             230             235             240

      ttg cgg gat tgg gtt aat cat gtc agg gaa aaa acg ggg aag gaa atg    1188
      Leu Arg Asp Trp Val Asn His Val Arg Glu Lys Thr Gly Lys Glu Met
                  245             250             255

      ttt acg gta gct gaa tat tgg cag aat gac ttg ggc gcg ctg gaa aac    1236
      Phe Thr Val Ala Glu Tyr Trp Gln Asn Asp Leu Gly Ala Leu Glu Asn
                  260             265             270

      tat ttg aac aaa aca aat ttt aat cat tca gtg ttt gac gtg ccg ctt    1284
      Tyr Leu Asn Lys Thr Asn Phe Asn His Ser Val Phe Asp Val Pro Leu
                  275             280             285

      cat tat cag ttc cat gct gca tcg aca cag gga ggc ggc tat gat atg    1332
      His Tyr Gln Phe His Ala Ala Ser Thr Gln Gly Gly Gly Tyr Asp Met
                  290             295             300
```

```
agg aaa ttg ctg aac ggt acg gtc gtt tcc aag cat ccg ttg aaa tcg        1380
Arg Lys Leu Leu Asn Gly Thr Val Val Ser Lys His Pro Leu Lys Ser
305                 310                 315                 320

gtt aca ttt gtc gat aac cat gat aca cag ccg ggg caa tcg ctt gag        1428
Val Thr Phe Val Asp Asn His Asp Thr Gln Pro Gly Gln Ser Leu Glu
                325                 330                 335

tcg act gtc caa aca tgg ttt aag ccg ctt gct tac gct ttt att ctc        1476
Ser Thr Val Gln Thr Trp Phe Lys Pro Leu Ala Tyr Ala Phe Ile Leu
                340                 345                 350

aca agg gaa tct gga tac cct cag gtt ttc tac ggg gat atg tac ggg        1524
Thr Arg Glu Ser Gly Tyr Pro Gln Val Phe Tyr Gly Asp Met Tyr Gly
                355                 360                 365

acg aaa gga gac tcc cag cgc gaa att cct gcc ttg aaa cac aaa att        1572
Thr Lys Gly Asp Ser Gln Arg Glu Ile Pro Ala Leu Lys His Lys Ile
        370                 375                 380

gaa ccg atc tta aaa gcg aga aaa cag tat gcg tac gga gca cag cat        1620
Glu Pro Ile Leu Lys Ala Arg Lys Gln Tyr Ala Tyr Gly Ala Gln His
385                 390                 395                 400

gat tat ttc gac cac cat gac att gtc ggc tgg aca agg gaa ggc gac        1668
Asp Tyr Phe Asp His His Asp Ile Val Gly Trp Thr Arg Glu Gly Asp
                405                 410                 415

agc tcg gtt gca aat tca ggt ttg gcg gca tta ata aca gac gga ccc        1716
Ser Ser Val Ala Asn Ser Gly Leu Ala Ala Leu Ile Thr Asp Gly Pro
                420                 425                 430

ggt ggg gca aag cga atg tat gtc ggc cgg caa aac gcc ggt gag aca        1764
Gly Gly Ala Lys Arg Met Tyr Val Gly Arg Gln Asn Ala Gly Glu Thr
                435                 440                 445

tgg cat gac att acc gga aac cgt tcg gag ccg gtt gtc atc aat tcg        1812
Trp His Asp Ile Thr Gly Asn Arg Ser Glu Pro Val Val Ile Asn Ser
        450                 455                 460

gaa ggc tgg gga gag ttt cac gta aac ggc ggg tcg gtt tca att tat        1860
Glu Gly Trp Gly Glu Phe His Val Asn Gly Gly Ser Val Ser Ile Tyr
465                 470                 475                 480

gtt caa aga tag aagagcagag aggacggatt tcctgaagga aatccgtttt          1912
Val Gln Arg

tttatttt                                                             1920


<210>  6
<211>  483
<212>  PRT
<213>  Bacillus licheniformis

<400>  6

Ala Asn Leu Asn Gly Thr Leu Met Gln Tyr Phe Glu Trp Tyr Met Pro
1               5                   10                  15

Asn Asp Gly Gln His Trp Arg Arg Leu Gln Asn Asp Ser Ala Tyr Leu
                20                  25                  30

Ala Glu His Gly Ile Thr Ala Val Trp Ile Pro Pro Ala Tyr Lys Gly
            35                  40                  45
```

Thr Ser Gln Ala Asp Val Gly Tyr Gly Ala Tyr Asp Leu Tyr Asp Leu
    50                  55              60

Gly Glu Phe His Gln Lys Gly Thr Val Arg Thr Lys Tyr Gly Thr Lys
65                  70              75                      80

Gly Glu Leu Gln Ser Ala Ile Lys Ser Leu His Ser Arg Asp Ile Asn
            85              90                      95

Val Tyr Gly Asp Val Val Ile Asn His Lys Gly Gly Ala Asp Ala Thr
            100             105             110

Glu Asp Val Thr Ala Val Glu Val Asp Pro Ala Asp Arg Asn Arg Val
        115             120             125

Ile Ser Gly Glu His Leu Ile Lys Ala Trp Thr His Phe His Phe Pro
    130             135             140

Gly Arg Gly Ser Thr Tyr Ser Asp Phe Lys Trp His Trp Tyr His Phe
145             150             155             160

Asp Gly Thr Asp Trp Asp Glu Ser Arg Lys Leu Asn Arg Ile Tyr Lys
            165             170             175

Phe Gln Gly Lys Ala Trp Asp Trp Glu Val Ser Asn Glu Asn Gly Asn
            180             185             190

Tyr Asp Tyr Leu Met Tyr Ala Asp Ile Asp Tyr Asp His Pro Asp Val
        195             200             205

Ala Ala Glu Ile Lys Arg Trp Gly Thr Trp Tyr Ala Asn Glu Leu Gln
    210             215             220

Leu Asp Gly Phe Arg Leu Asp Ala Val Lys His Ile Lys Phe Ser Phe
225             230             235             240

Leu Arg Asp Trp Val Asn His Val Arg Glu Lys Thr Gly Lys Glu Met
            245             250             255

Phe Thr Val Ala Glu Tyr Trp Gln Asn Asp Leu Gly Ala Leu Glu Asn
        260             265             270

Tyr Leu Asn Lys Thr Asn Phe Asn His Ser Val Phe Asp Val Pro Leu
    275             280             285

His Tyr Gln Phe His Ala Ala Ser Thr Gln Gly Gly Gly Tyr Asp Met
    290             295             300

Arg Lys Leu Leu Asn Gly Thr Val Val Ser Lys His Pro Leu Lys Ser
305             310             315             320

```
Val Thr Phe Val Asp Asn His Asp Thr Gln Pro Gly Gln Ser Leu Glu
              325                 330                 335

Ser Thr Val Gln Thr Trp Phe Lys Pro Leu Ala Tyr Ala Phe Ile Leu
              340                 345                 350

Thr Arg Glu Ser Gly Tyr Pro Gln Val Phe Tyr Gly Asp Met Tyr Gly
          355                 360                 365

Thr Lys Gly Asp Ser Gln Arg Glu Ile Pro Ala Leu Lys His Lys Ile
    370                 375                 380

Glu Pro Ile Leu Lys Ala Arg Lys Gln Tyr Ala Tyr Gly Ala Gln His
385                 390                 395                 400

Asp Tyr Phe Asp His His Asp Ile Val Gly Trp Thr Arg Glu Gly Asp
              405                 410                 415

Ser Ser Val Ala Asn Ser Gly Leu Ala Ala Leu Ile Thr Asp Gly Pro
              420                 425                 430

Gly Gly Ala Lys Arg Met Tyr Val Gly Arg Gln Asn Ala Gly Glu Thr
          435                 440                 445

Trp His Asp Ile Thr Gly Asn Arg Ser Glu Pro Val Val Ile Asn Ser
    450                 455                 460

Glu Gly Trp Gly Glu Phe His Val Asn Gly Gly Ser Val Ser Ile Tyr
465                 470                 475                 480

Val Gln Arg
```

```
<210>    7
<211>    1776
<212>    DNA
<213>    Bacillus sp.


<220>
<221>    CDS
<222>    (145)..(1692)

<220>
<221>    sig_peptide
<222>    (145)..(238)

<220>
<221>    mat_peptide
<222>    (238)..()

<400>    7
atataaattt gaaatgaaca cctatgaaaa tatggtagcg attgcgcgac gagaaaaaac        60

ttgggagtta ggaagtgata ttaaaggatt ttttttgact tgttgtgaaa acgcttgcat       120

aaattgaagg agagggtgct tttt atg aaa ctt cat aac cgt ata att agc          171
```

```
                              Met Lys Leu His Asn Arg Ile Ile Ser
                              -30                     -25

     gta cta tta aca cta ttg tta gct gta gct gtt ttg ttt cca tat atg    219
     Val Leu Leu Thr Leu Leu Leu Ala Val Ala Val Leu Phe Pro Tyr Met
             -20             -15                 -10

     acg gaa cca gca caa gcc cat cat aat ggg acg aat ggg acc atg atg    267
     Thr Glu Pro Ala Gln Ala His His Asn Gly Thr Asn Gly Thr Met Met
         -5              -1  1               5                   10

     cag tat ttt gaa tgg cat ttg cca aat gac ggg aac cac tgg aac agg    315
     Gln Tyr Phe Glu Trp His Leu Pro Asn Asp Gly Asn His Trp Asn Arg
                     15              20                  25

     tta cga gat gac gca gct aac tta aag agt aaa ggg att acc gct gtt    363
     Leu Arg Asp Asp Ala Ala Asn Leu Lys Ser Lys Gly Ile Thr Ala Val
                 30              35                  40

     tgg att cct cct gca tgg aag ggg act tcg caa aat gat gtt ggg tat    411
     Trp Ile Pro Pro Ala Trp Lys Gly Thr Ser Gln Asn Asp Val Gly Tyr
             45              50                  55

     ggt gcc tat gat ttg tac gat ctt ggt gag ttt aac caa aag gga acc    459
     Gly Ala Tyr Asp Leu Tyr Asp Leu Gly Glu Phe Asn Gln Lys Gly Thr
         60              65                  70

     gtc cgt aca aaa tat ggc aca agg agt cag ttg caa ggt gcc gtg aca    507
     Val Arg Thr Lys Tyr Gly Thr Arg Ser Gln Leu Gln Gly Ala Val Thr
     75              80                  85                  90

     tct ttg aaa aat aac ggg att caa gtt tat ggg gat gtc gtg atg aat    555
     Ser Leu Lys Asn Asn Gly Ile Gln Val Tyr Gly Asp Val Val Met Asn
                     95              100                 105

     cat aaa ggt gga gca gac ggg aca gag atg gta aat gcg gtg gaa gtg    603
     His Lys Gly Gly Ala Asp Gly Thr Glu Met Val Asn Ala Val Glu Val
                 110             115                 120

     aac cga agc aac cga aac caa gaa ata tca ggt gaa tac acc att gaa    651
     Asn Arg Ser Asn Arg Asn Gln Glu Ile Ser Gly Glu Tyr Thr Ile Glu
             125             130                 135

     gca tgg acg aaa ttt gat ttc cct gga aga gga aat acc cat tcc aac    699
     Ala Trp Thr Lys Phe Asp Phe Pro Gly Arg Gly Asn Thr His Ser Asn
         140             145                 150

     ttt aaa tgg cgc tgg tat cat ttt gat ggg aca gat tgg gat cag tca    747
     Phe Lys Trp Arg Trp Tyr His Phe Asp Gly Thr Asp Trp Asp Gln Ser
     155             160                 165                 170

     cgt cag ctt cag aac aaa ata tat aaa ttc aga ggt acc gga aag gca    795
     Arg Gln Leu Gln Asn Lys Ile Tyr Lys Phe Arg Gly Thr Gly Lys Ala
                     175             180                 185

     tgg gac tgg gaa gta gat ata gag aac ggc aac tat gat tac ctt atg    843
     Trp Asp Trp Glu Val Asp Ile Glu Asn Gly Asn Tyr Asp Tyr Leu Met
                 190             195                 200

     tat gca gac att gat atg gat cat cca gaa gta atc aat gaa ctt aga    891
     Tyr Ala Asp Ile Asp Met Asp His Pro Glu Val Ile Asn Glu Leu Arg
                 205             210                 215

     aat tgg gga gtt tgg tat aca aat aca ctt aat cta gat gga ttt aga    939
     Asn Trp Gly Val Trp Tyr Thr Asn Thr Leu Asn Leu Asp Gly Phe Arg
         220             225                 230

     atc gat gct gtg aaa cat att aaa tac agc tat acg aga gat tgg cta    987
     Ile Asp Ala Val Lys His Ile Lys Tyr Ser Tyr Thr Arg Asp Trp Leu
```

```
          235                    240                    245                    250
    aca cat gtg cgt aac acc aca ggt aaa cca atg ttt gca gtt gca gaa    1035
    Thr His Val Arg Asn Thr Thr Gly Lys Pro Met Phe Ala Val Ala Glu
                    255                260                265

    ttt tgg aaa aat gac ctt gct gca atc gaa aac tat tta aat aaa aca    1083
    Phe Trp Lys Asn Asp Leu Ala Ala Ile Glu Asn Tyr Leu Asn Lys Thr
                    270                275                280

    agt tgg aat cac tcc gtg ttc gat gtt cct ctt cat tat aat ttg tac    1131
    Ser Trp Asn His Ser Val Phe Asp Val Pro Leu His Tyr Asn Leu Tyr
                    285                290                295

    aat gca tct aat agt ggt ggc tat ttt gat atg aga aat att tta aat    1179
    Asn Ala Ser Asn Ser Gly Gly Tyr Phe Asp Met Arg Asn Ile Leu Asn
            300                305                310

    ggt tct gtc gta caa aaa cac cct ata cat gca gtc aca ttt gtt gat    1227
    Gly Ser Val Val Gln Lys His Pro Ile His Ala Val Thr Phe Val Asp
    315                320                325                330

    aac cat gac tct cag cca gga gaa gca ttg gaa tcc ttt gtt caa tcg    1275
    Asn His Asp Ser Gln Pro Gly Glu Ala Leu Glu Ser Phe Val Gln Ser
                    335                340                345

    tgg ttc aaa cca ctg gca tat gca ttg att ctg aca agg gag caa ggt    1323
    Trp Phe Lys Pro Leu Ala Tyr Ala Leu Ile Leu Thr Arg Glu Gln Gly
                    350                355                360

    tac cct tcc gta ttt tac ggt gat tac tac ggt ata cca act cat ggt    1371
    Tyr Pro Ser Val Phe Tyr Gly Asp Tyr Tyr Gly Ile Pro Thr His Gly
                    365                370                375

    gtt cct tcg atg aaa tct aaa att gat cca ctt ctg cag gca cgt caa    1419
    Val Pro Ser Met Lys Ser Lys Ile Asp Pro Leu Leu Gln Ala Arg Gln
            380                385                390

    acg tat gcc tac gga acc caa cat gat tat ttt gat cat cat gat att    1467
    Thr Tyr Ala Tyr Gly Thr Gln His Asp Tyr Phe Asp His His Asp Ile
    395                400                405                410

    atc ggc tgg acg aga gaa ggg gac agc tcc cac cca aat tca gga ctt    1515
    Ile Gly Trp Thr Arg Glu Gly Asp Ser Ser His Pro Asn Ser Gly Leu
                    415                420                425

    gca act att atg tcc gat ggg cca ggg ggt aat aaa tgg atg tat gtc    1563
    Ala Thr Ile Met Ser Asp Gly Pro Gly Gly Asn Lys Trp Met Tyr Val
                    430                435                440

    ggg aaa cat aaa gct ggc caa gta tgg aga gat atc acc gga aat agg    1611
    Gly Lys His Lys Ala Gly Gln Val Trp Arg Asp Ile Thr Gly Asn Arg
                    445                450                455

    tct ggt acc gtc acc att aat gca gat ggt tgg ggg aat ttc act gta    1659
    Ser Gly Thr Val Thr Ile Asn Ala Asp Gly Trp Gly Asn Phe Thr Val
            460                465                470

    aac gga ggg gca gtt tcg gtt tgg gtg aag caa taaataagga acaagaggcg    1712
    Asn Gly Gly Ala Val Ser Val Trp Val Lys Gln
    475                480                485

    aaaattactt tcctacatgc agagctttcc gatcactcat acacccaata taaattggaa    1772

    gctt                                                                1776


    <210>  8
    <211>  516
```

<212> PRT
<213> Bacillus sp.

<400> 8

Met Lys Leu His Asn Arg Ile Ile Ser Val Leu Leu Thr Leu Leu Leu
    -30                 -25             -20

Ala Val Ala Val Leu Phe Pro Tyr Met Thr Glu Pro Ala Gln Ala His
-15                 -10             -5                 -1  1

His Asn Gly Thr Asn Gly Thr Met Met Gln Tyr Phe Glu Trp His Leu
            5               10              15

Pro Asn Asp Gly Asn His Trp Asn Arg Leu Arg Asp Asp Ala Ala Asn
        20              25              30

Leu Lys Ser Lys Gly Ile Thr Ala Val Trp Ile Pro Pro Ala Trp Lys
    35              40              45

Gly Thr Ser Gln Asn Asp Val Gly Tyr Gly Ala Tyr Asp Leu Tyr Asp
50              55              60              65

Leu Gly Glu Phe Asn Gln Lys Gly Thr Val Arg Thr Lys Tyr Gly Thr
            70              75              80

Arg Ser Gln Leu Gln Gly Ala Val Thr Ser Leu Lys Asn Asn Gly Ile
            85              90              95

Gln Val Tyr Gly Asp Val Val Met Asn His Lys Gly Gly Ala Asp Gly
        100             105             110

Thr Glu Met Val Asn Ala Val Glu Val Asn Arg Ser Asn Arg Asn Gln
    115             120             125

Glu Ile Ser Gly Glu Tyr Thr Ile Glu Ala Trp Thr Lys Phe Asp Phe
130             135             140             145

Pro Gly Arg Gly Asn Thr His Ser Asn Phe Lys Trp Arg Trp Tyr His
            150             155             160

Phe Asp Gly Thr Asp Trp Asp Gln Ser Arg Gln Leu Gln Asn Lys Ile
            165             170             175

Tyr Lys Phe Arg Gly Thr Gly Lys Ala Trp Asp Trp Glu Val Asp Ile
        180             185             190

Glu Asn Gly Asn Tyr Asp Tyr Leu Met Tyr Ala Asp Ile Asp Met Asp
    195             200             205

His Pro Glu Val Ile Asn Glu Leu Arg Asn Trp Gly Val Trp Tyr Thr
210             215             220             225

```
Asn Thr Leu Asn Leu Asp Gly Phe Arg Ile Asp Ala Val Lys His Ile
            230             235             240

Lys Tyr Ser Tyr Thr Arg Asp Trp Leu Thr His Val Arg Asn Thr Thr
            245             250             255

Gly Lys Pro Met Phe Ala Val Ala Glu Phe Trp Lys Asn Asp Leu Ala
            260             265             270

Ala Ile Glu Asn Tyr Leu Asn Lys Thr Ser Trp Asn His Ser Val Phe
    275             280             285

Asp Val Pro Leu His Tyr Asn Leu Tyr Asn Ala Ser Asn Ser Gly Gly
290             295             300             305

Tyr Phe Asp Met Arg Asn Ile Leu Asn Gly Ser Val Val Gln Lys His
            310             315             320

Pro Ile His Ala Val Thr Phe Val Asp Asn His Asp Ser Gln Pro Gly
            325             330             335

Glu Ala Leu Glu Ser Phe Val Gln Ser Trp Phe Lys Pro Leu Ala Tyr
            340             345             350

Ala Leu Ile Leu Thr Arg Glu Gln Gly Tyr Pro Ser Val Phe Tyr Gly
    355             360             365

Asp Tyr Tyr Gly Ile Pro Thr His Gly Val Pro Ser Met Lys Ser Lys
370             375             380             385

Ile Asp Pro Leu Leu Gln Ala Arg Gln Thr Tyr Ala Tyr Gly Thr Gln
            390             395             400

His Asp Tyr Phe Asp His His Asp Ile Ile Gly Trp Thr Arg Glu Gly
            405             410             415

Asp Ser Ser His Pro Asn Ser Gly Leu Ala Thr Ile Met Ser Asp Gly
    420             425             430

Pro Gly Gly Asn Lys Trp Met Tyr Val Gly Lys His Lys Ala Gly Gln
    435             440             445

Val Trp Arg Asp Ile Thr Gly Asn Arg Ser Gly Thr Val Thr Ile Asn
450             455             460             465

Ala Asp Gly Trp Gly Asn Phe Thr Val Asn Gly Gly Ala Val Ser Val
            470             475             480

Trp Val Lys Gln
            485
```

<210> 9

```
<211>  40
<212>  DNA
<213>  Artificial sequence

<220>
<223>  Primer DAX-8N

<400>  9
gctgcggccg ctgcagatgg mttgaayggw acgatgatgc                              40


<210>  10
<211>  43
<212>  DNA
<213>  Artificial sequence

<220>
<223>  Primer DAX-8C

<400>  10
ggccgtcgac ttattggttc acrtacacgg atacagatcc tcc                         43


<210>  11
<211>  32
<212>  DNA
<213>  Artificial sequence

<220>
<223>  Primer Amrk752-E84Q

<400>  11
ctaaggcaca gcttcaacga gctattgggt cc                                      32


<210>  12
<211>  35
<212>  DNA
<213>  Artificial sequence

<220>
<223>  Primer Amrk752-N96D

<400>  12
ccttaaatct aatgatatcg atgtatacgg agatg                                  35


<210>  13
<211>  34
<212>  DNA
<213>  Artificial sequence

<220>
<223>  Primer Amrk752-A315S

<400>  13
ttataatttt taccggtctt cacaacaagg tgga                                   34


<210>  14
<211>  32
<212>  DNA
<213>  Artificial sequence

<220>
<223>  Primer Amrk752-A445V

<400>  14
gtaggacgtc agaatgtagg acaaacatgg ac                                      32
```

```
<210>  15
<211>  35
<212>  DNA
<213>  Artificial sequence

<220>
<223>  Primer Amrk752-G464N

<400>  15
ccgttacaat taataacgat ggatggggcg aattc                                35


<210>  16
<211>  34
<212>  DNA
<213>  Artificial sequence

<220>
<223>  Primer Amrk230-N121D

<400>  16
gcaagctgtt caagtagatc caacgaatcg ttgg                                34


<210>  17
<211>  34
<212>  DNA
<213>  Artificial sequence

<220>
<223>  Primer Amrk230-N390H

<400>  17
gcttgatgca cgtcaagatt acgcatatgg cacg                                34
```